# EUROPEAN PATENT SPECIFICATION

(11) **EP 3 064 500 B1**
(45) Date of publication and mention of the grant of the patent: **25.08.2021**
(21) Application number: 14858489.9
(22) Date of filing: 17.10.2014
(51) Int. Cl.: H01L 51/46, C07D 487/04, C07D 519/00, C07D 495/04

(54) **SINGLE MOLECULE AND ORGANIC SOLAR CELL COMPRISING SAME**
EINZELMOLEKÜL UND ORGANISCHE SOLARZELLE DAMIT
MOLÉCULE UNIQUE ET CELLULE SOLAIRE ORGANIQUE LA COMPRENANT

(30) Priority: 28.10.2013 KR 20130128237
(43) Date of publication of application: 07.09.2016
(73) Proprietor: LG Chem, Ltd., Seoul 150-721 (KR)
(72) Inventor: CHO, Keun, Daejeon 305-738 (KR); LEE, Jaechol, Daejeon 305-738 (KR); BAE, Jaesoon, Daejeon 305-738 (KR); LEE, Jiyoung, Daejeon 305-738 (KR); KIM, Jinseck, Daejeon 305-738 (KR); CHOI, Doo Whan, Daejeon 305-738 (KR)
(74) Representative: Hoffmann Eitle
(86) International application number: PCT/KR2014/009791
(87) International publication number: WO 2015/064937

(56) References cited:
- EP-A2- 2 034 537
- WO-A1-2010/049323
- WO-A1-2013/030325
- WO-A2-2009/047104
- CN-A- 103 087 083
- BRIGHT WALKER, ET AL.: "Optimization of energy levels by molecular design:evaluation of bis-diketopyrrolopyrrole molecular donormaterials for bulk heterojunction solar cells", ENERGY ENVIRON. SCI., vol. 6, 10 January 2013 (2013-01-10), pages 952-962, XP002770489,
- YUKI SUNA ET AL.: 'Ambipolar Behavior of Hydrogen-Bonded Diketopyrrolopyrro le-Thiophene Co-oligomers Formed from Their Soluble Precursors' ORGANIC LETTERS vol. 14, 2012, pages 3356 - 3359, XP055339608
- None

## Description

### [Technical Field]

This application claims priority to Korean Patent Application No. 10-2013-0128237, filed with the Korean Intellectual Property Office on October 28, 2013.

The present disclosure relates to a single molecule and an organic solar cell including the same.

### [Background Art]

An organic solar cell is a device capable of directly converting solar energy to electric energy by applying a photovoltaic effect. Solar cells are divided into inorganic solar cells and organic solar cells depending on the materials forming a thin film. Typical solar cells are fabricated using a p-n junction by doping crystalline silicon (Si), an inorganic semiconductor. Electrons are holes generated by light absorption spread to p-n junction points, are accelerated by the electric field, and move to an electrode. Power conversion efficiency of this process is defined as a ratio of power given to an external circuit and solar power put into a solar cell, and the ratio has been accomplished up to 24% when measured under a currently standardized hypothetical solar irradiation condition. However, existing inorganic solar cells already has a limit in economic feasibility and material supplies, and therefore, organic material semiconductor solar cells that are easily processed, inexpensive and have various functionality have been highly favored as a long-term alternative energy source.

For solar cells, it is important to increase efficiency so as to output as much electric energy as possible from solar energy. In order to improve efficiency of such solar cells, generating as much excitons as possible inside a semiconductor is important, however, taking the generated charges outside without loss is also important. One of the reasons for the charge loss is the dissipation of the generated electrons and holes by recombination. Various methods for delivering the generated electrons or holes without loss have been proposed, however, most of the methods require additional processes, and accordingly, the fabricating costs may increase.

### [Prior Art Documents]

### [Non-Patent Documents]

Two-layer organic photovoltaic cell (C. W. Tang, Appl. Phys. Lett., 48, 183 (1996));
Efficiencies via Network of Internal Donor-Acceptor Heterojunctions (G. Yu, J. Gao, J. C. Hummelen, F. Wudl, A. J. Heeger, Science, 270, 1789 (1995));
"Optimization of energy levels by molecular design: evaluation of bis-diketopyrrolopyrrole molecular donor materials for bulk heterojunction solar cells" (B. Walker et al., Energy & Environmental Science, 6, 952 (2013)) discloses donor materials for bulk heterojunction solar cells consisting of two diketopyrrolopyrrole (DPP) cores with different aromatic π-bridges between the DPP units and different end-capping groups.

### [Patent Documents]

CN 103 087 083 A describes a thiophene-substituted pyrrolopyrroledione derivative as a solar cell material and a method for preparing the material.

WO 2013/030325 A1 discloses compositions comprising a diketopyrrolopyrrole oligomer and a polymeric material, and their use as organic semiconductor in organic devices such as solar cells.

### [Disclosure]

### [Technical Problem]

An object of the present specification is to provide a single molecule and an organic solar cell including the same.

### [Technical Solution]

The present specification provides a single molecule which is represented by any of Chemical Formulae 10, 11, 15, 16 and 18 to 21: 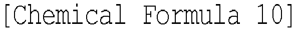 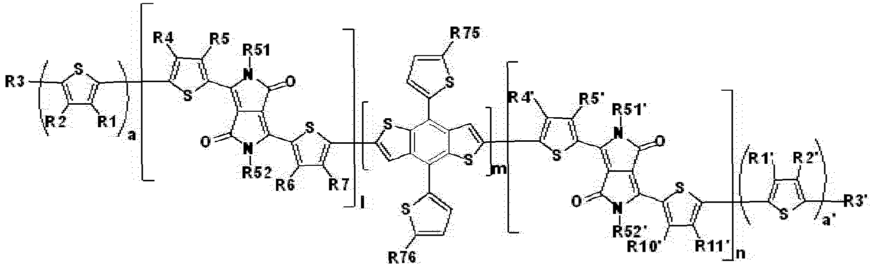 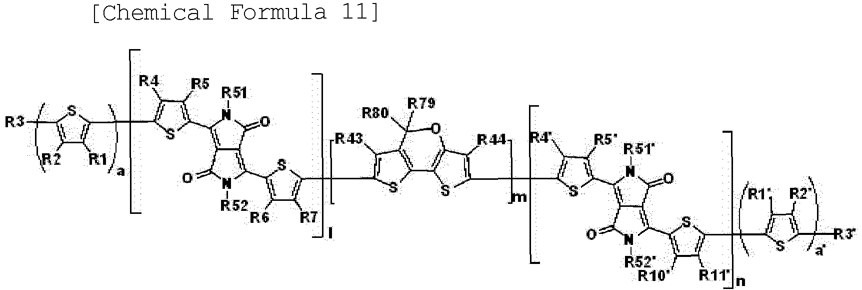 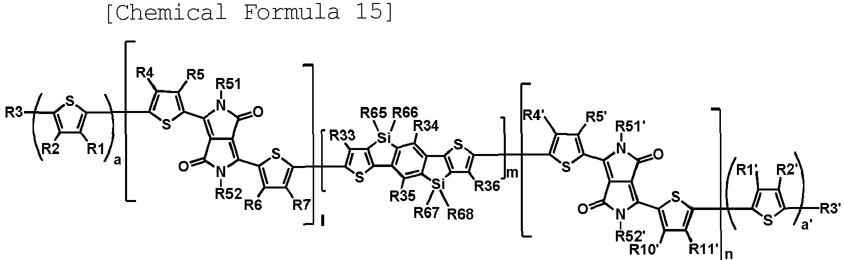 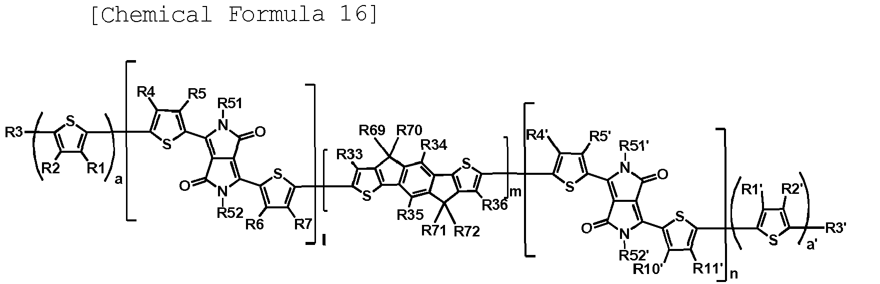 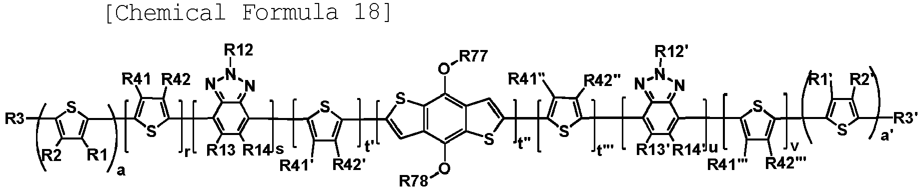 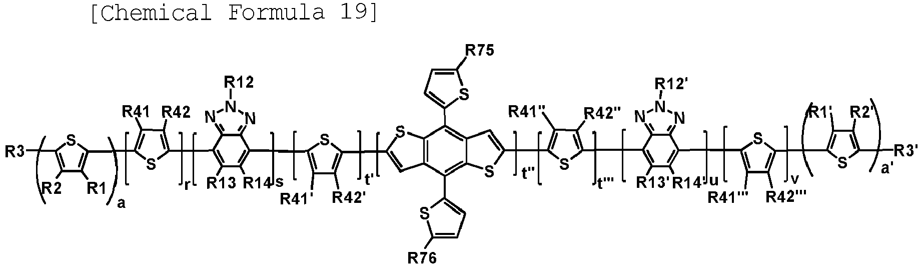 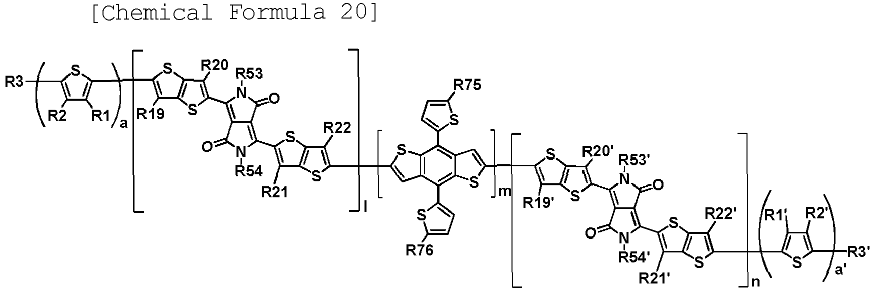 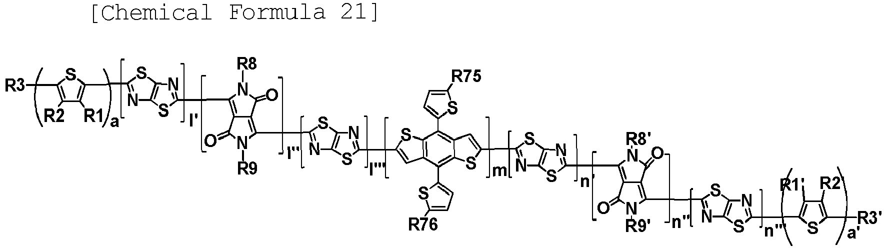 wherein, in Chemical Formulae 10, 11, 15, 16 and 18 to 21:
a is an integer of 1 to 5, and when a is an integer of 2 to 5, the two or more structures in the parenthesis are the same as or different from each other,
R1 and R2 are the same as or different from each other, and each independently selected from the group consisting of hydrogen; deuterium; a halogen group; a nitrile group; a nitro group; an imide group; an amide group; a hydroxyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; and a substituted or unsubstituted heteroring group,
R3 is hydrogen; a substituted or unsubstituted alkyl group; or any one of the following structures, 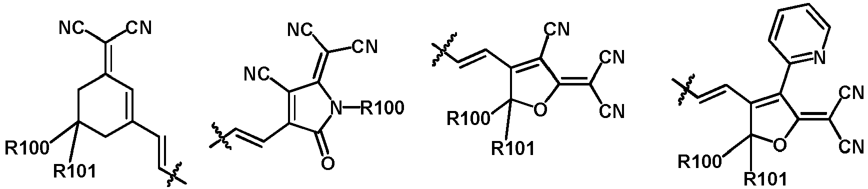 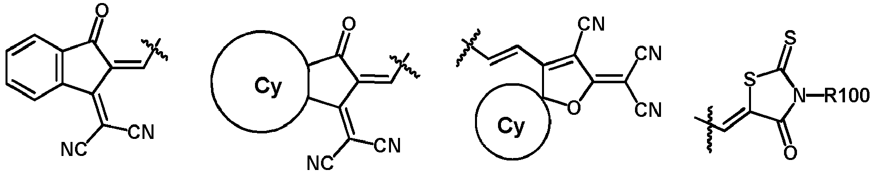 in the structure,
Cy is a substituted or unsubstituted aryl group; or a substituted or unsubstituted heteroring group,
R1 to R9, R12 to R14, R19 to R22, R33 to R36, R41 to R44, R51 to R54, R65 to R72, R75 to R80, R1' to R5', R8' to R14', R19' to R22', R41' to R42', R51' to R54', R41" to R42", R41''' to R42''', and R100, R101 are the same as or different from each other, and each independently selected from the group consisting of hydrogen; deuterium; a halogen group; a nitrile group; a nitro group; an imide group; an amide group; a hydroxyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; and a substituted or unsubstituted heteroring group, and
a' has the same definition as a;
1 is a repetition number of units, and 0 ≤ l ≤ 10;
m is a repetition number of units, and 0 ≤ m ≤ 10;
n is a repetition number of units, and 0 ≤ n ≤ 10;
l+n ≥ 2;
2 ≤ l+m+n ≤ 10;
l'+l"+l'"= l;
n'+n"+n'"= n;
t'+t"+t'"=t;
r is a repetition number of units, and 0 ≤ r ≤ 10;
s is a repetition number of units, and 0 ≤ s ≤ 10;
t is a repetition number of units, and 0 ≤ t ≤ 10;
u is a repetition number of units, and 2 ≤ u ≤ 10;
v is a repetition number of units, and 0 ≤ v ≤ 10;
s+u ≥ 2; and
2 ≤ r+s+t+u+v ≤ 10;
when l, m, n, p, r, s, t, u and v each are two or more, the two or more structures of [ ] are the same as or different from each other.

In addition, the present specification provides an organic solar cell including a first electrode; a second electrode provided opposite to the first electrode; and one or more organic material layers provided between the first electrode and the second electrode, and including a photoactive layer, wherein one or more layers of the organic material layers include the single molecule described above.

### [Advantageous Effects]

A single molecule of the present specification may be used as a material of an organic material layer of an organic solar cell, and the organic solar cell using the single molecule may exhibit excellent properties in an open voltage increase, an efficiency increase and the like. Particularly, the single molecule according to one embodiment of the present specification has a deep HOMO level, a small band gap, and high charge mobility, therefore, may exhibit excellent properties. The single molecule according to one embodiment of the present specification may be used either alone or as a mixture with other materials in an organic solar cell, may improve efficiency, and may improve the lifespan properties of a device by thermal stability of the compound.

### [Description of Drawings]

FIG. 1 is a diagram showing an NMR spectrum of Monomer 1 prepared in Example 1.
FIG. 2 is a diagram showing an NMR spectrum of Monomer 2 prepared in Example 2.
FIG. 3 is a diagram showing an NMR spectrum of Monomer 3' prepared in Example 3.
FIG. 4 is a diagram showing an MS spectrum of Monomer 3' prepared in Example 3.
FIG. 5 is a diagram showing an NMR spectrum of Monomer 4' prepared in Example 4.
FIG. 6 is a diagram showing an MS spectrum of Monomer 4' prepared in Example 4.
FIG. 7 is a diagram showing an NMR spectrum of Monomer 5' prepared in Example 5.
FIG. 8 is a diagram showing an MS spectrum of Monomer 5' prepared in Example 5.
FIG. 9 is a diagram showing an NMR spectrum of Monomer 6' prepared in Example 6.
FIG. 10 is a diagram showing an NMR spectrum of Monomer 7 prepared in Example 7.
FIG. 11 is a diagram showing an NMR spectrum of a compound prepared in Example 8.
FIG. 12 is a diagram showing an MS spectrum of a compound prepared in Example 8.
FIG. 13 is a diagram showing an MS spectrum of a compound prepared in Example 9.
FIG. 14 is a diagram showing an MS spectrum of a compound prepared in Example 10.
FIG. 15 is a diagram showing an MS spectrum of a compound prepared in Example 11.
FIG. 16 is a diagram showing an MS spectrum of a compound prepared in Example 12.
FIG. 17 is a diagram showing an NMR spectrum of Compound E according to Example 13.
FIG. 18 is a diagram showing an MS spectrum of Single molecule 1 prepared in Example 14.
FIG. 19 is a diagram showing an MS spectrum of Single molecule 3 prepared in Example 16.
FIG. 20 is a diagram showing an MS spectrum of Single molecule 4 prepared in Example 17.
FIG. 21 is a diagram showing an MS spectrum of Single molecule 5 prepared in Example 18.
FIG. 22 is a diagram showing an MS spectrum of Single molecule 6 prepared in Example 19.
FIG. 23 is a diagram showing an MS spectrum of Single molecule 7 prepared in Example 20.
FIG. 24 is a diagram showing a UV-Vis spectrum of Single molecule 1 prepared in Example 14.
FIG. 25 is a diagram showing an electrochemical measurement result (cyclic voltametry) of Single molecule 1 prepared in Example 14.
FIG. 26 is a diagram showing high performance liquid chromatography (HPLC) of Compound 7-1 prepared in Example 20.
FIG. 27 shows a UV-Vis absorption spectrum of Compound 7-1 of Example 20 in a chlorobenzene solution and in a film phase in which the chlorobenzene solution is heat treated.
FIG. 28 is a diagram showing an MS spectrum of Compound 7-2 of Example 20.
FIG. 29 is a diagram showing an electrochemical measurement result (cyclic voltametry) of Compound 7-2 of Example 20.
FIG. 30 is a diagram showing current density by voltage of an organic solar cell including Single molecule 1 prepared in Example 14.
FIG. 31 is a diagram showing current density by voltage of an organic solar cell including Single molecule 2 prepared in Example 15.
FIG. 32 is a diagram showing current density by voltage of an organic solar cell including Single molecule 3 prepared in Example 16.
FIG. 33 is a diagram showing current density by voltage of an organic solar cell including Single molecule 4 prepared in Example 17.
FIG. 34 is a diagram showing current density by voltage of an organic solar cell including Single molecule 5 prepared in Example 18.
FIG. 35 is a diagram showing current density by voltage of an organic solar cell including Single molecule 6 prepared in Example 19.
FIG. 36 is a diagram showing current density by voltage of an organic solar cell including Single molecule 7 prepared in Example 20.
FIG. 37 is a diagram showing current density by voltage of an organic solar cell according to Comparative Example 1.
FIG. 38 is a diagram showing a laminated structure of an organic solar cell according to one embodiment of the present specification.

### [Reference Numeral]

- 101:: Substrate
- 102:: First Electrode
- 103:: Hole Transfer Layer
- 104:: Photoactive Layer
- 105:: Second Electrode

### [Mode for Disclosure]

Hereinafter, the present specification will be described in detail.

A 'unit' in the present specification is a structure included in a single molecule, and means a structure in which a monomer binds in a single molecule by polymerization.

'Including a unit' in the present specification means being included in a single molecule.

Examples of the substituents are described below, however, the substituents are not limited thereto.

In one embodiment of the present specification, the purity of the single molecule is 90% to 99.99%.

In the present specification, 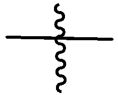 means a site linking to the main chain of the single molecule or a site linking to other substituents.

A term "substituted or unsubstituted" in the present specification means being substituted with one, two or more substituents selected from the group consisting of deuterium; a halogen group; an alkyl group; an alkenyl group; an alkoxy group; a cycloalkyl group; a silyl group; an arylalkenyl group; an aryloxy group; an alkylthioxy group; an alkylsulfoxy group; an arylsulfoxy group; a boron group; an alkylamine group; an aralkylamine group; an arylamine group; a heteroaryl group; a carbazole group; an arylamine group; an aryl group; a nitrile group; a nitro group; a hydroxyl group and a heteroring group, or being substituted with a substituent linking two or more substituents among the substituents illustrated above, or having no substituents. For example, "a substituent linking two or more substituents" may include a biphenyl group. In other words, a biphenyl group may be an aryl group, or interpreted as a substituent linking two phenyl groups. the term means being substituted with a substituent linking two or more substituents among the substituents illustrated above. For example, "a substituent linking two or more substituents" may include a biphenyl group. In other words, a biphenyl group may be an aryl group, or interpreted as a substituent linking two phenyl groups.

The term "substitution" means a hydrogen atom bonding to a carbon atom of a compound is changed to another substituent, and the position of substitution is not limited as long as it is a position at which a hydrogen atom is substituted, that is, a position at which a substituent may substitute, and when two or more substituents substitute, the two or more substituents may be the same as or different from each other.

In the present specification, the number of carbon atoms of the imide group is not particularly limited, but is preferably 1 to 25. Specifically, compounds having the following structures may be included, but the compound is not limited thereto.

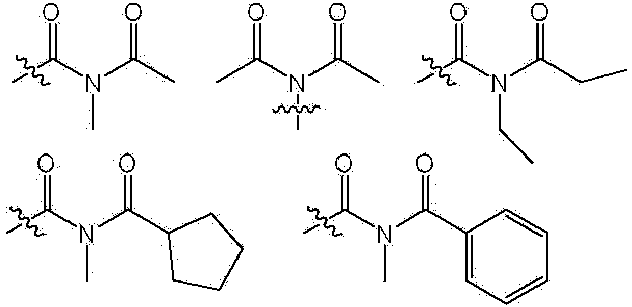

In the present specification, in the amide group, the nitrogen of the amide group may be once or twice substituted with hydrogen, a linear, branched or cyclic alkyl group having 1 to 25 carbon atoms, or an aryl group having 6 to 25 carbon atoms. Specifically, compounds having the following structural formulae may be included, but the compound is not limited thereto.

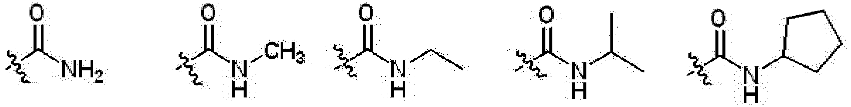

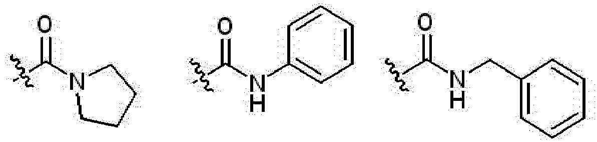

In the present specification, the alkyl group may be linear or branched, and the number of carbon atoms is not particularly limited, but is preferably 1 to 50. Specific examples thereof include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl and the like, but are not limited thereto.

In the present specification, the cycloalkyl group is not particularly limited, but preferably has 3 to 60 carbon atoms. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl and the like, but are not limited thereto.

In the present specification, the alkoxy group may be linear, branched or cyclic. The number of carbon atoms of the alkoxy group is not particularly limited, but is preferably 1 to 20. Specific examples thereof may include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the alkenyl group may be linear or branched, and although not particularly limited thereto, the number of carbon atoms is preferably 2 to 40. Specific examples thereof may include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group and the like, but are not limited thereto.

In the present specification, the aryl group may be a monocyclic aryl group or a multicyclic aryl group, and includes a case in which an alkyl group having 1 to 25 carbon atoms or an alkoxy group having 1 to 25 carbon atoms is substituted. In addition, the aryl group in the present specification may mean an aromatic ring.

When the aryl group is a monocyclic aryl group, the number of carbon atoms is not particularly limited, but is preferably 6 to 25. Specific examples of the monocyclic aryl group may include a phenyl group, a biphenyl group, a terphenyl and the like, but are not limited thereto.

When the aryl group is a multicyclic aryl group, the number of carbon atoms is not particularly limited, but is preferably 10 to 24. Specific example of the multicyclic aryl group may include a naphthyl group, an anthracenyl group, a phenanthryl group, a pyrenyl group, a perylenyl group, a crycenyl group, a fluorenyl group and the like, but are not limited thereto.

In the present specification, the fluorenyl group has a structure in which two cyclic organic compounds are linked through one atom.

In the present specification, the fluorenyl group may be substituted, and adjacent substituents may bond to each other to form a ring.

When the fluorenyl group is substituted, 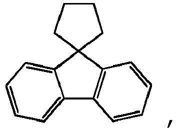 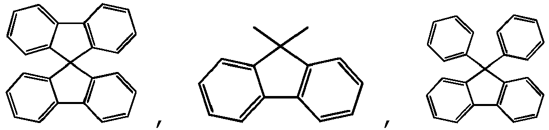 and the like may be included. However, the structure is not limited thereto.

In the present specification, the silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present specification, the number of carbon atoms of the amine group is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, a phenylnaphthylamine group, a ditolylamine group, a phenyltolylamine group, a triphenylamine group and the like, but are not limited thereto.

In the present specification, examples of the arylamine group include a substituted or unsubstituted monoarylamine group, a substituted or unsubstituted diarylamine group, or a substituted or unsubstituted triarylamine group. The aryl group in the arylamine group may be a monocyclic aryl group or a multicyclic aryl group. The arylamine group including two or more aryl groups may include monocyclic aryl groups, multicyclic aryl groups, or monocyclic aryl groups and multicyclic aryl groups at the same time.

Specific examples of the arylamine group include phenylamine, naphthylamine, biphenylamine, anthracenylamine, 3-methyl-phenylamine, 4-methyl-naphthylamine, 2-methyl-biphenylamine, 9-methyl-anthracenylamine, a diphenylamine group, a phenylnaphthylamine group, a ditolylamine group, a phenyltolylamine group, carbazole, a triphenylamine group and the like, but are not limited thereto.

In the present specification, the heteroring group includes one or more atoms that are not carbon, which are heteroatoms, and specifically, the heteroatom may include one or more atoms selected from the group consisting of O, N, Se, S and the like. The number of carbon atoms of the heteroring group is not particularly limited, but is preferably 2 to 60. Examples of the heteroring group include a thiophene group, a furan group, a pyrrole group, an imidazole group, a thiazole group, an oxazole group, an oxadiazole group, a triazole group, a pyridyl group, a bipyridyl group, a pyrimidyl group, a triazine group, a triazole group, an acridyl group, a pyridazine group, a pyrazinyl group, a qinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, a dibenzofuranyl group and the like, but are not limited thereto.

In the present specification, the aryl group in the aryloxy group, the arylthioxy group, the arylsulfoxy group and the aralkylamine group is the same as the examples of the aryl group described above. Specific examples of the aryloxy group include phenoxy, p-tolyloxy, m-tolyloxy, 3,5-dimethyl-phenoxy, 2,4,6-trimethylphenoxy, p-tert-butylphenoxy, 3-biphenyloxy, 4-biphenyloxy, 1-naphthyloxy, 2-naphthyloxy, 4-methyl-1-naphthyloxy, 5-methyl-2-naphthyloxy, 1-anthryloxy, 2-anthryloxy, 9-anthryloxy, 1-phenanthryloxy, 3-phenanthryloxy, 9-phenanthryloxy and the like, examples of the arylthioxy group include a phenylthioxy group, a 2-methylphenylthioxy group, a 4-tert-butylphenylthioxy group and the like, and examples of the arylsulfoxy group include a benzenesulfoxy group, a p-toluenesulfoxy group and the like, but the examples are not limited thereto.

In the present specification, the heteroaryl group in the heteroarylamine group may be selected from the examples of the heteroring group described above.

In the present specification, the alkyl group in the alkylthioxy group and the alkylsulfoxy group is the same as the examples of the alkyl group described above. Specific examples of the alkylthioxy group include a methylthioxy group, an ethylthioxy group, a tert-butylthioxy group, a hexylthioxy group, an octylthioxy group and the like, and examples of the alkylsulfoxy group include a methylsulfoxy group, an ethylsulfoxy group, a propylsulfoxy group, a butylsulfoxy group and the like, but the examples are not limited thereto.
[

In the present specification, the single molecule is represented by any one of the following Chemical Formula 10, 11, 15, 16 and 18 to 21.

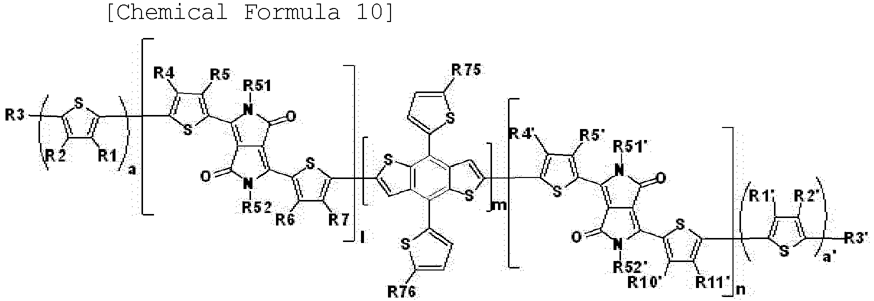

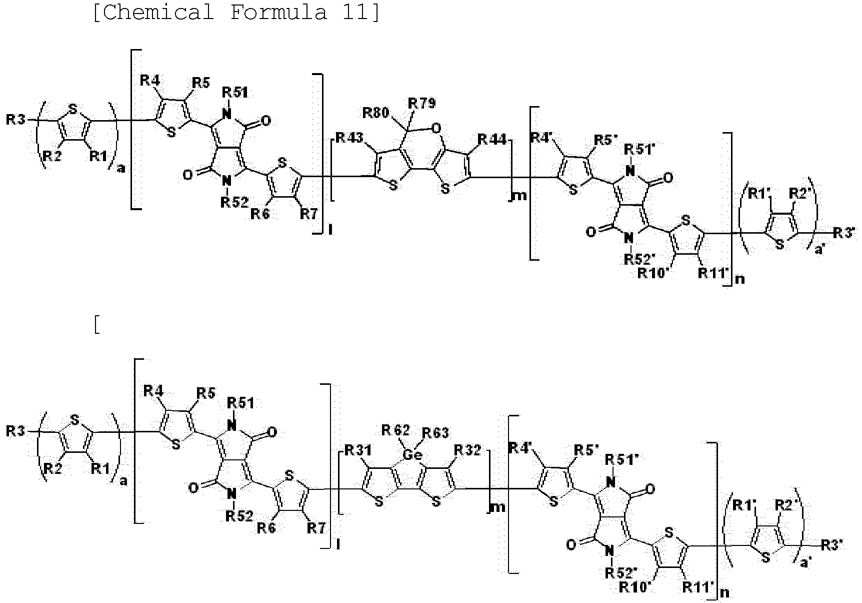

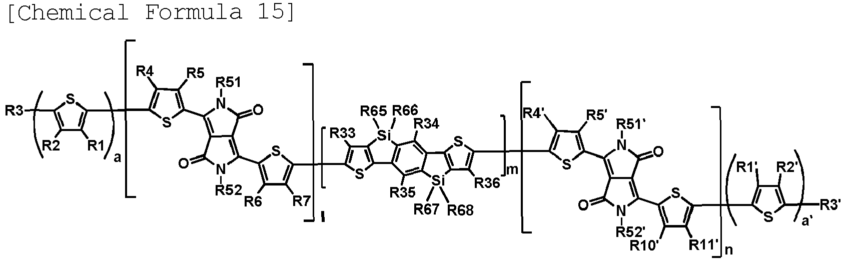

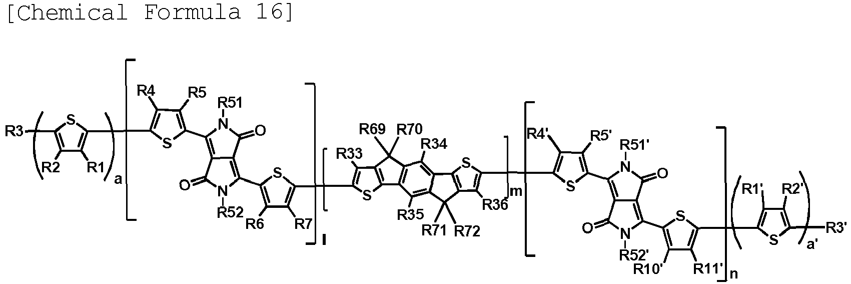

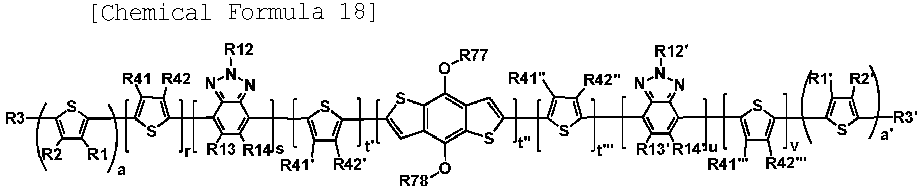

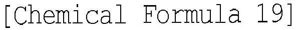

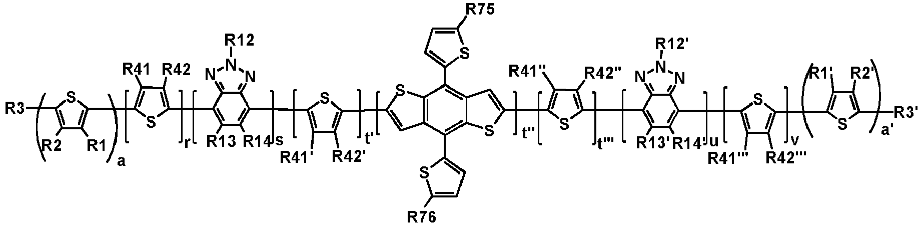

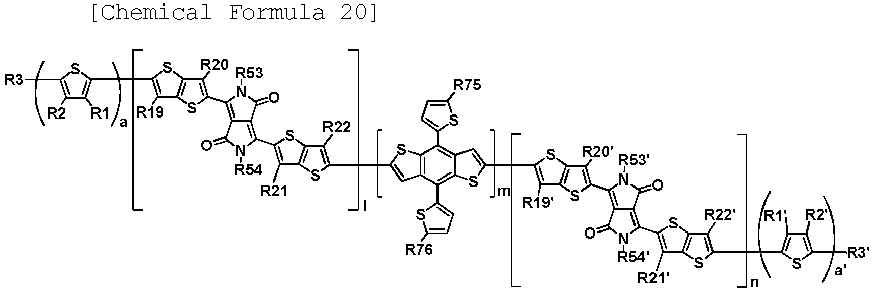

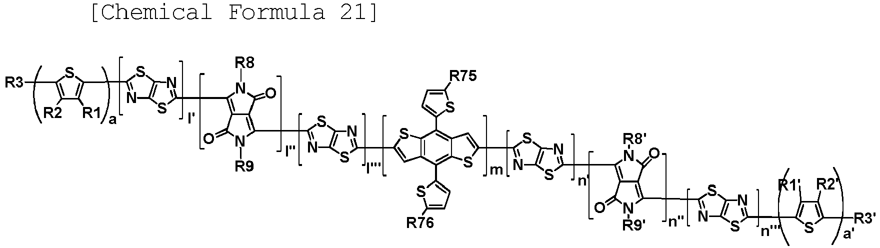

In Chemical Formulae 10, 11, 15, 16 and 18 to 21,
a is an integer of 1 to 5, and when a is an integer of 2 to 5, the two or more structures in the parenthesis are the same as or different from each other;
a' has the same definition as a,
R1 to R9, R12 to R14, R19 to R22, R33 to R36, R41 to R44, R51 to R54, R65 to R72, R75 to R80, R1' to R5', R8' to R14', R19' to R22', R41' to R42', R51' to R54', R41" to R42" and R41''' to R42''' are the same as or different from each other, and each independently hydrogen; deuterium; a halogen group; a nitrile group; a nitro group; an imide group; an amide group; a hydroxyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; or a substituted or unsubstituted heteroring group,
1 is a repetition number of units, and 0 ≤ l ≤ 10,
m is a repetition number of units, and 0 ≤ m ≤ 10,
n is a repetition number of units, and 0 ≤ n ≤ 10,
l+n ≥2,
2 ≤ l+m+n ≤ 10,
l'+l"+l'"= l,
n'+n"+n'"=n,
p is a repetition number of units, and 2 ≤ p ≤ 10,
r is a repetition number of units, and 0 ≤ r ≤ 10,
s is a repetition number of units, and 0 ≤ s ≤ 10,
t is a repetition number of units, and 0 ≤ t ≤ 10,
u is a repetition number of units, and 2 ≤ u ≤ 10,
v is a repetition number of units, and 0 ≤ v ≤ 10,
s+u ≥ 2,
2 ≤ r+s+t+u+v ≤ 10,
t'+t"+t'" = t, and
when l, m, n, p, r, s, t, u and v each are two or more, the two or more structures of [ ] are the same as or different from each other.

In one embodiment of the present specification, the single molecule may be any one of the following single molecules.

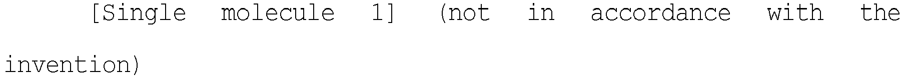

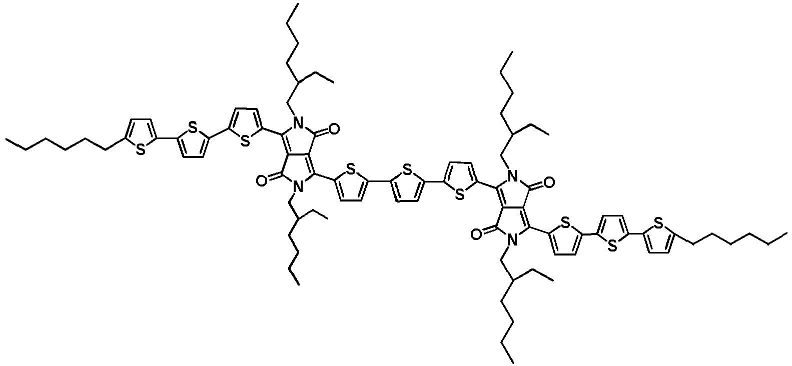

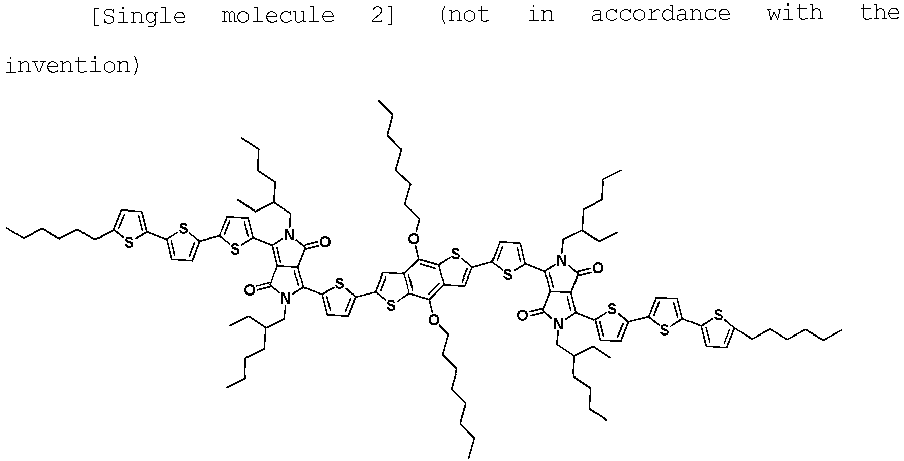

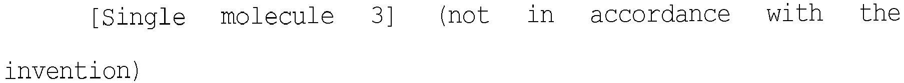

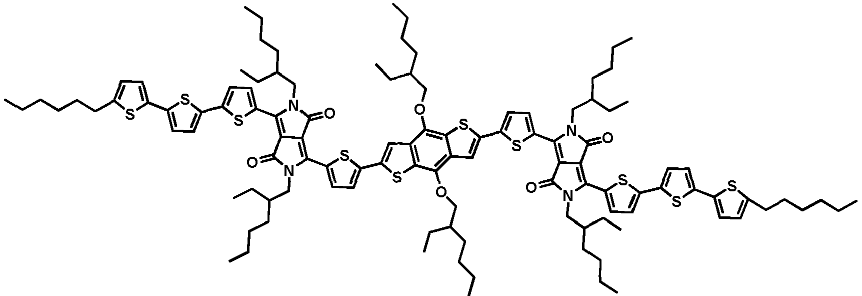

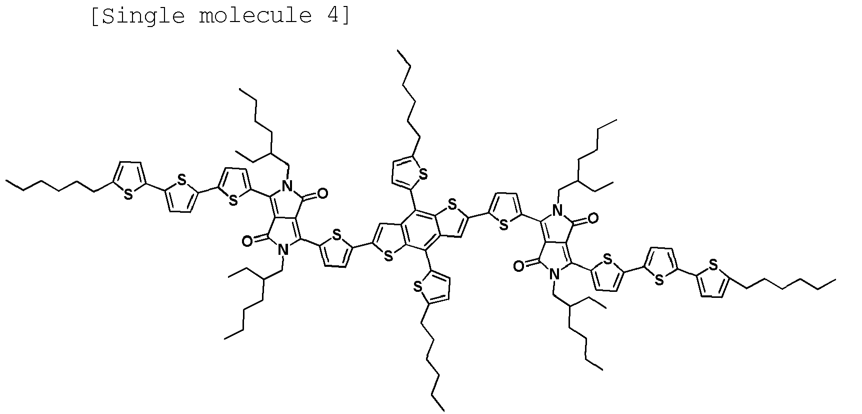

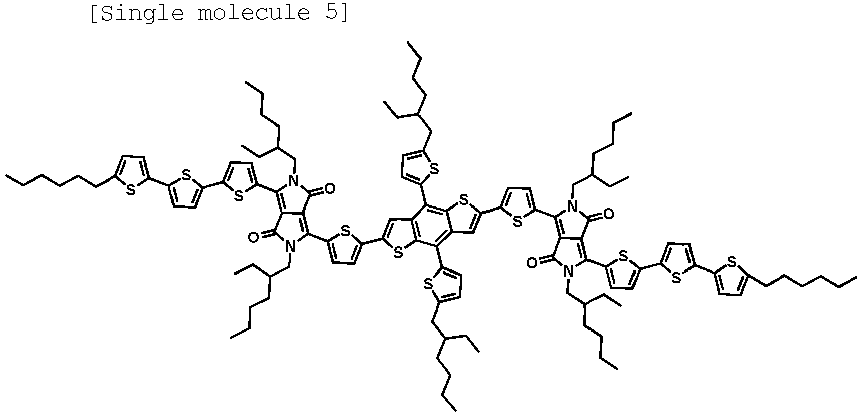

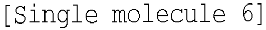

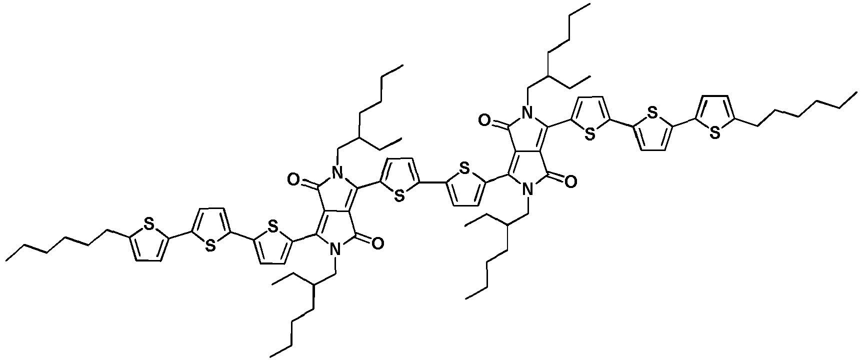

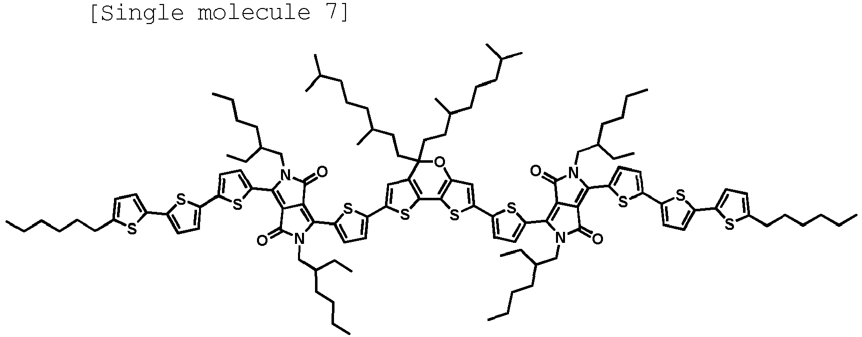

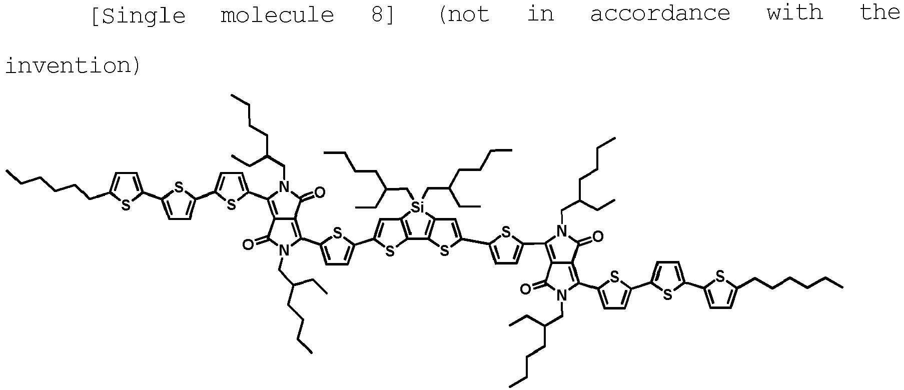

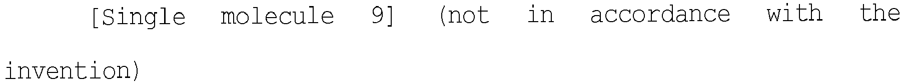

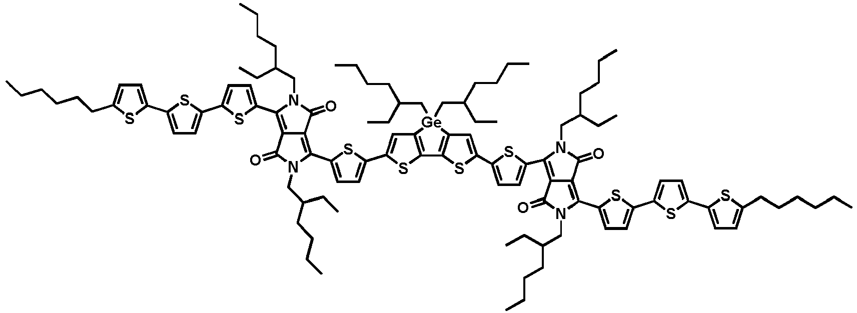

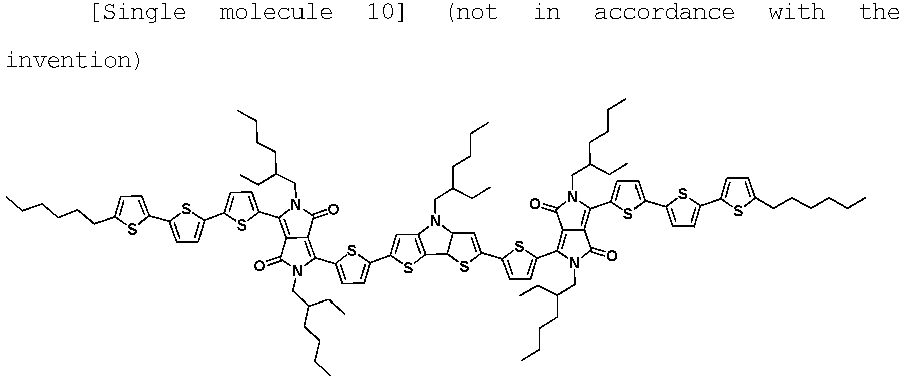

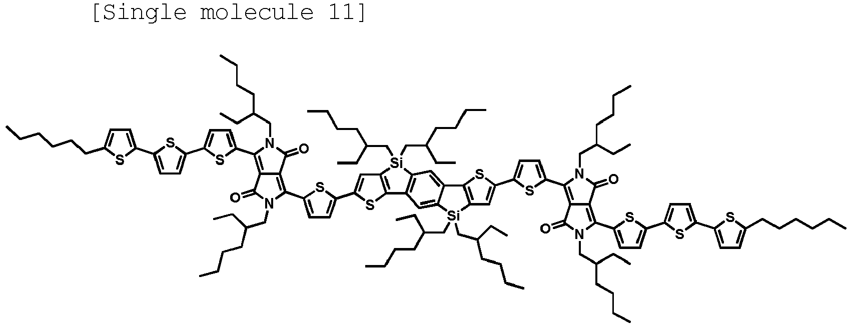

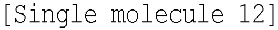

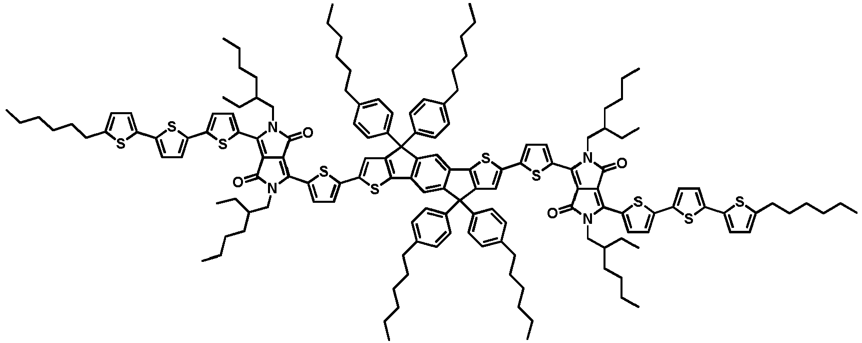

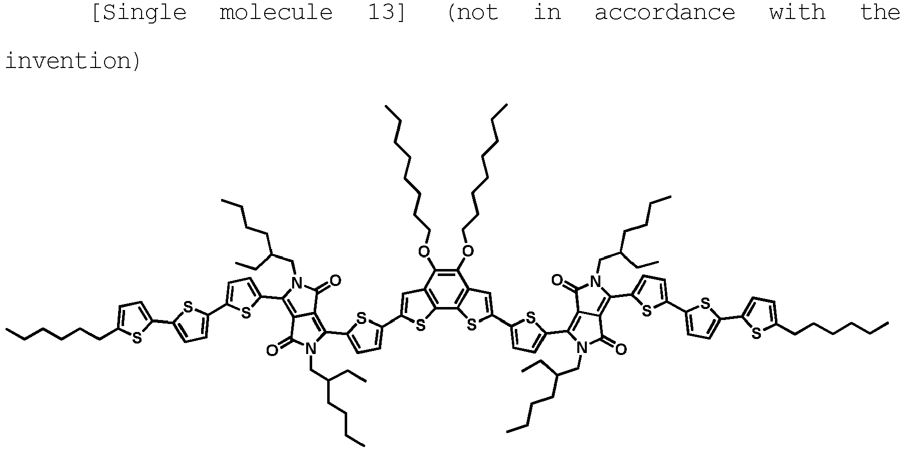

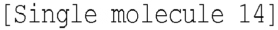

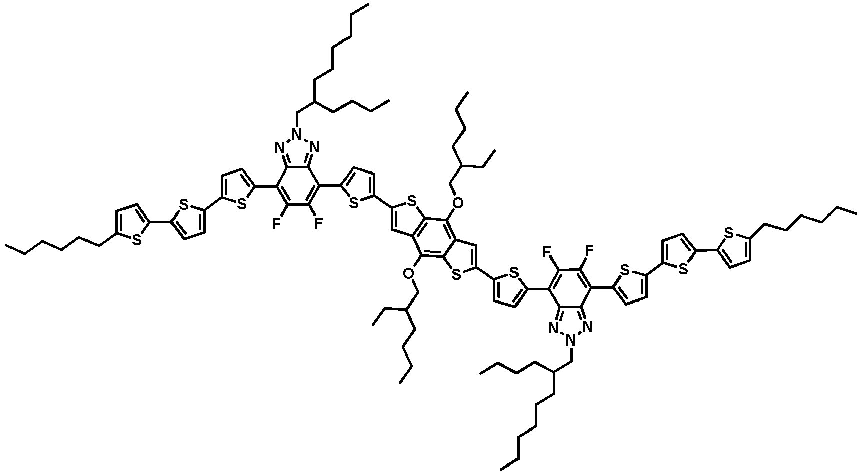

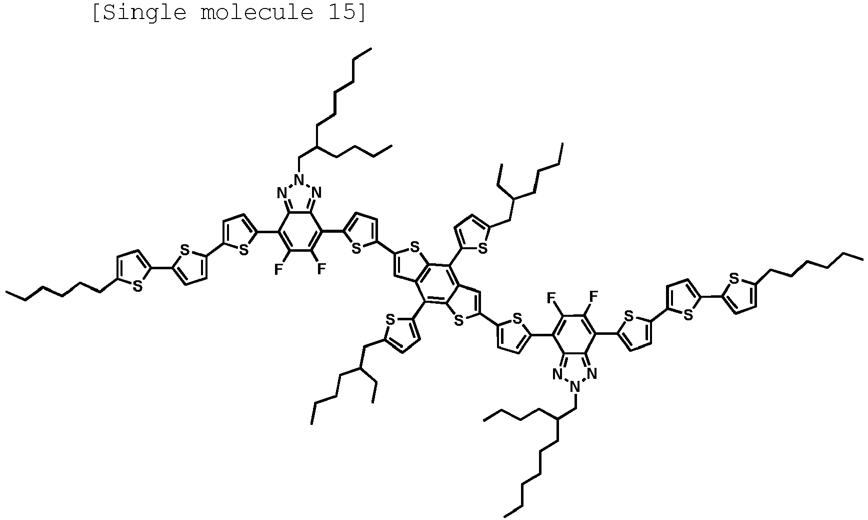

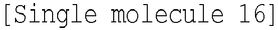

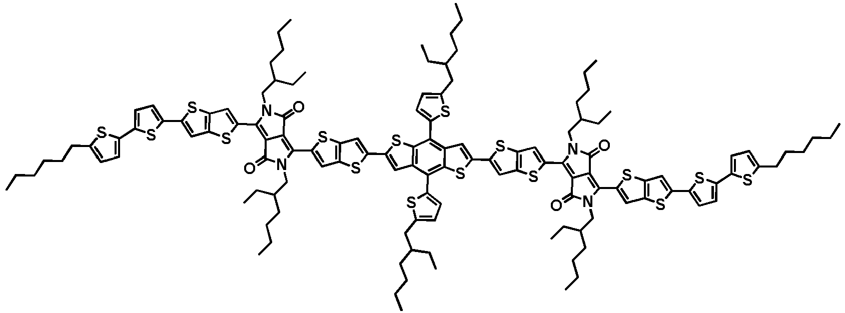

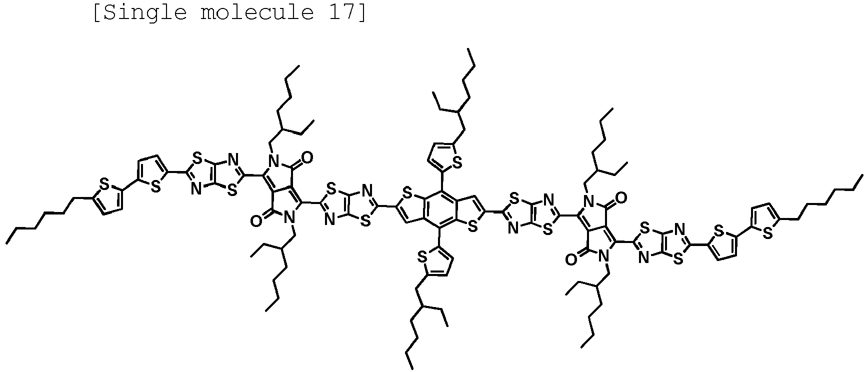

In one embodiment of the present specification, the molecular weight of the single molecule is 100 g/mol to 20,000 g/mol. In another embodiment, the molecular weight of the single molecule is 500 g/mol to 10,000 g/mol.

The single molecule may be prepared based on the preparation examples described below.

The single molecule according to the present specification may be prepared using a multi-step chemical reaction. After monomers are prepared through an alkylation reaction, a Grignard reaction, a Suzuki coupling reaction, a Stille coupling reaction and the like, final single molecules may be prepared through a carbon-carbon coupling reaction such as a Stille coupling reaction. When a substituent to introduce is a boronic acid or a boronic ester compound, a Suzuki coupling reaction may be used, and when a substituent to introduce is a tributyltin compound, a Stille coupling reaction may be used, however, the method is not limited thereto.

One embodiment of the present specification provides an organic solar cell including a first electrode; a second electrode provided opposite to the first electrode; and one or more organic material layers provided between the first electrode and the second electrode and including a photoactive layer, wherein one or more layers of the organic material layer include the single molecule.

An organic solar cell according to one embodiment of the present invention includes a first electrode, a photoactive layer and a second electrode. The organic solar cell may further include a substrate, a hole transfer layer and/or an electron transfer layer.

In one embodiment of the present specification, when the organic solar cell receives photons from an external light source, electrons and holes are generated between an electron donor and an electron acceptor. The generated holes are transferred to an anode through the electron donor layer.

In one embodiment of the present specification, the organic material layer includes a hole transfer layer, a hole injection layer, or a layer carrying out hole transfer and hole injection at the same time, and the hole transfer layer, the hole injection layer, or the layer carrying out hole transfer and hole injection at the same time includes the single molecule.

In another embodiment, the organic material layer includes an electron injection layer, an electron transfer layer, or a layer carrying out electron injection and electron transfer at the same time, and the electron injection layer, the electron transfer layer, or the layer carrying out electron injection and electron transfer at the same time includes the single molecule.

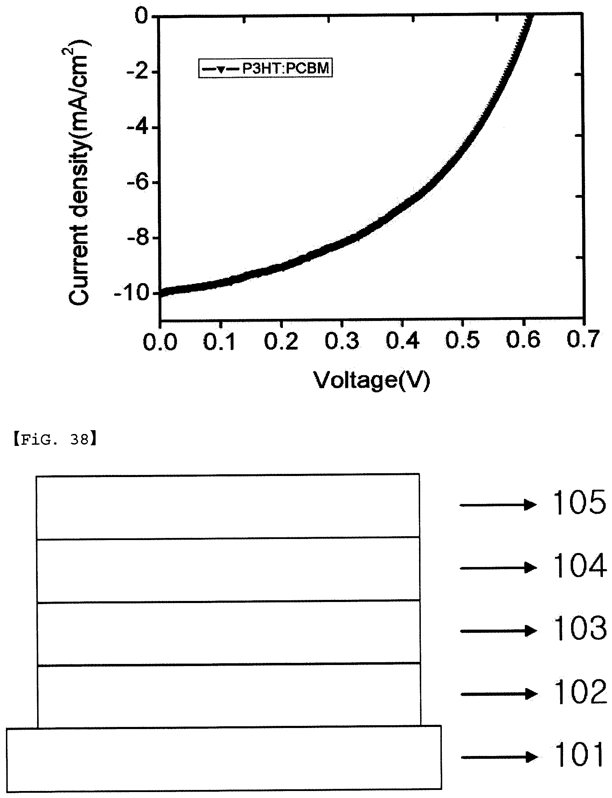

FIG. 38 is a diagram showing a laminated structure of an organic solar cell according to one embodiment of the present specification.

In one embodiment of the present specification, when the organic solar cell receives photons from an external light source, electrons and holes are generated between an electron donor and an electron acceptor. The generated holes are transferred to an anode through the electron donor layer.

In one embodiment of the present specification, the organic solar cell may further include additional organic material layers. The organic solar cell may reduce the number of organic material layers by using an organic material having various functions at the same time.

In one embodiment of the present specification, the first electrode is an anode, and the second electrode is a cathode. In another embodiment, the first electrode is a cathode, and the second electrode is an anode.

In one embodiment of the present specification, the organic solar cell may have a structure in which a cathode, a photoactive layer and an anode are arranged in consecutive order, or may have a structure in which an anode, a photoactive layer and a cathode are arranged in consecutive order, however, the structure is not limited thereto.

In another embodiment, the organic solar cell may have a structure in which an anode, a hole transfer layer, a photoactive layer, an electron transfer layer and a cathode are arranged in consecutive order, or may have a structure in which a cathode, an electron transfer layer, a photoactive layer, a hole transfer layer and an anode are arranged in consecutive order, however, the structure is not limited thereto.

In one embodiment of the present specification, the organic solar cell has a normal structure.

In one embodiment of the present specification, the organic solar cell has an inverted structure.

In one embodiment of the present specification, the organic solar cell has a tandem structure.

The organic solar cell according to one embodiment of the present invention may have one, two or more photoactive layers.

In another embodiment, a buffer layer may be provided between a photoactive layer and a hole transfer layer, or between a photoactive layer and an electron transfer layer. Herein, a hole transfer layer may be further provided between an anode and a hole transfer layer. In addition, an electron transfer layer may be further provided between a cathode and an electron transfer layer.

In one embodiment of the present specification, the photoactive layer includes one, two or more selected from the group consisting of an electron donor and an electron acceptor, and the electron donor material includes the single molecule.

In one embodiment of the present specification, the electron acceptor material may be selected from the group consisting of fullerene, fullerene derivatives, bathocuproine, semiconductor elements, semiconductor compounds, and a combination thereof. Specifically, one, two or more selected from the group consisting of fullerene, fullerene derivatives ((6,6)-phenyl-C₆₁-butyric acid-methylester (PCBM) or (6,6)-phenyl-C₆₁-butyric acid-cholesteryl ester (PCBCR), perylene, polybenzimidazole (PBI), and 3,4,9,10-perylene-tetracarboxylic bis-benzimidazole (PTCBI) may be included, however, the electron acceptor material is not limited thereto.

In one embodiment of the present specification, the electron donor and the electron acceptor form a bulk heterojunction (BHJ). The electron donor material and the electron acceptor material are mixed in a ratio (w/w) of 1:10 to 10:1.

A Bulk heterojunction means an electron donor material and an electron acceptor material being mixed together in a photoactive layer.

In one embodiment of the present specification, the photoactive layer has a bilayer structure including an n-type organic material layer and a p-type organic material layer, and the p-type organic material layer includes the single molecule.

The substrate in the present specification may include a glass substrate or a transparent plastic substrate, which has excellent transparency, surface smoothness, handling easiness and water resistance, but is not limited thereto, and substrates typically used for organic solar cells may be used without limit. Specific examples thereof include glass, polyethylene terphthalate (PET), polyethylene naphthalate (PEN), polypropylene (PP), polyimide (PI), triacetyl cellulose (TAC) and the like, but are not limited thereto.

The anode electrode may include a material that is transparent and has excellent conductivity, but the material is not limited thereto. Examples of the anode material include metals such as vanadium, chromium, copper, zinc or gold, or alloys thereof; metal oxides such as zinc oxides, indium oxides, indium tin oxides (ITO) or indium zinc oxides (IZO); and a combination of metals and oxides such as ZnO:Al or SnO₂:Sb; conductive polymers such as poly (3-methylthiophene), poly[3,4-(ethylene-1,2-dioxy)thiophene] (PEDOT), polypyrrole and polyaniline, and the like, but are not limited thereto.

The method of forming the anode electrode is not particularly limited, however, the anode electrode may be formed by being applied to one surface of a substrate or coated in the form of a film using a method such as sputtering, E-beam, thermal deposition, spin coating, screen printing, ink jet printing, doctor blade or gravure printing.

When the anode electrode is formed on a substrate, the result may go through processes of cleaning, dehydrating and modifying to be hydrophilic.

For example, after a patterned ITO substrate is cleaned with a cleaning agent, acetone and isopropyl alcohol (IPA) in consecutive order, the ITO substrate is dried for 1 to 30 minutes at 100 to 150°C, preferably for 10 minutes at 120°C, on a heating plate in order to remove moisture, and when the substrate is completely cleaned, the surface of the substrate is modified to be hydrophilic.

Through the surface modification described above, the junctional surface potential may be maintained at a level suitable for the surface potential of a photoactive layer. In addition, when a surface is modified, a polymer thin film may be readily formed on an anode electrode, and the quality of the thin film may be improved.

Preprocessing technologies for an anode electrode include
a) a surface oxidation method using parallel plate discharge,
b) a method of oxidizing the surface through the ozone generated by UV rays in a vacuum, and c) an oxidation method using the oxygen radicals generated by plasma.

One of the methods described above may be selected depending on the condition of an anode electrode or a substrate. However, it is commonly preferable to prevent the leave of oxygen on the surface of an anode electrode or a substrate and to suppress the remaining of moisture and organic materials as much as possible, no matter which method is used. In this case, practical effects of the preprocessing may be maximized.

As a specific example, a method of oxidizing the surface through the ozone generated by UV rays may be used. Herein, a patterned ITO substrate may be fully dried by baking the patterned ITO substrate on a hot plate after being ultrasonic cleaned, and the patterned ITO substrate is introduced into a chamber and then may be cleaned by the ozone generated by reacting oxygen gas with UV light using a UV lamp.

However, the method of surface modification of the patterned ITO substrate in the present specification is not particularly limited, and any method oxidizing a substrate may be used.

The cathode electrode may include a metal having small work function, but is not limited thereto. Specific examples thereof may include metals such as magnesium, calcium, sodium, potassium, titanium, indium, yttrium, lithium, gadolinium, aluminum, silver, tin and lead, or alloys thereof; or multilayer structure materials such as LiF/Al, LiO₂/Al, LiF/Fe, Al:Li, Al:BaF₂ and Al:BaF₂:Ba, but are not limited thereto.

The cathode electrode may be formed by being deposited inside a thermal depositor having a vacuum degree of 5x10⁻⁷ torr or less, but the formation is not limited to this method.

The hole transfer layer and/or the electron transfer layer material play a role of efficiently transferring the electrons and the holes separated in a photoactive layer to an electrode, and the material is not particularly limited.

The hole transfer layer material may include PEDOT:PSS (Poly(3,4-ethylenediocythiophene) doped with poly(styrenesulfonic acid)), molybdenum oxides (MoOₓ); vanadium oxides (V₂O₅); nickel oxides (NiO); tungsten oxides (WOₓ), and the like, but is not limited thereto.

The electron transfer layer material may include electron-extracting metal oxides, and may specifically include a metal complex of 8-hydroxyquinoline; a complex including Alq₃; a metal complex including Liq; LiF; Ca; titanium oxides (TiOₓ); zinc oxides (ZnO); cesium carbonate (Cs₂CO₃), and the like, but is not limited thereto.

The photoactive layer may be formed by dissolving a photoactive material such as an electron donor and/or an electron acceptor in an organic solvent, and then applying the solution using a method such as spin coating, dip coating, screen printing, spray coating, doctor blade and brush painting, but the method is not limited thereto.

Hereinafter, a method for preparing the single molecule and a method for fabricating an organic solar cell including the single molecule will be described in detail with reference to the following preparation examples and examples. However, the following examples are for illustrative purposes only, and the scope of the present specification is not limited thereto.

### Example 1. Monomer Synthesis-1

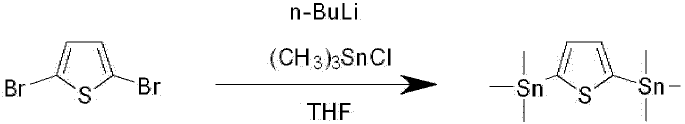

After 2,5-dibromothiophene (9.68 g, 40.0 mmol) was placed and dissolved in 200 ml of tetrahydrofuran (THF), the temperature was lowered to -78°C. 1.6 M n-butyllithium (n-BuLi) in hexane (55 ml, 88 mmol) was slowly added thereto at this temperature, and the result was stirred for 1 hour. After that, 1 M trimethyltinchloride in THF (100 ml, 100 mmol) was added thereto at once, the temperature was raised to room temperature, and the result was stirred for 12 hours. This solution was poured into ice, extracted three times using diethyl ether, washed three times with water, and residual water was removed using magnesium sulfate (MgSO₄). The remaining solution was vacuumed to remove the solvent, and recrystallized with methanol to obtain a white solid.
Yield: 73.1%

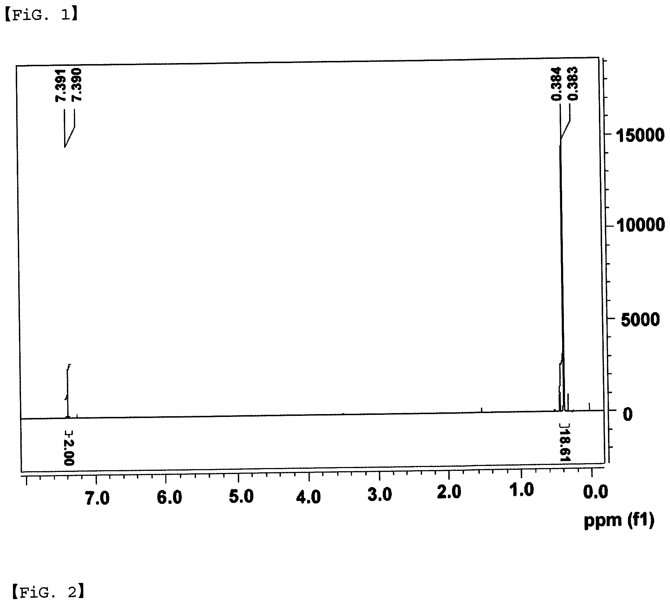

FIG. 1 is a diagram showing an NMR spectrum of the compound obtained in Example 1.

### Example 2. Monomer Synthesis-2 (Synthesis of 5-(tri-n-butylstannyl)-5'-hexyl-2,2'-bithiophene)

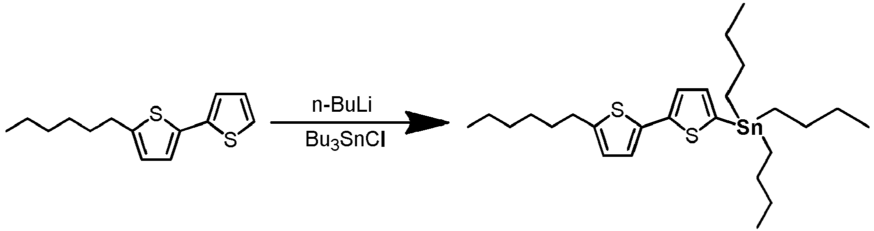

Monomer 2 was synthesized using a method described in the Journal of Polymer Science Part A: Polymer Chemistry, 49, 1155-1162 (2011).

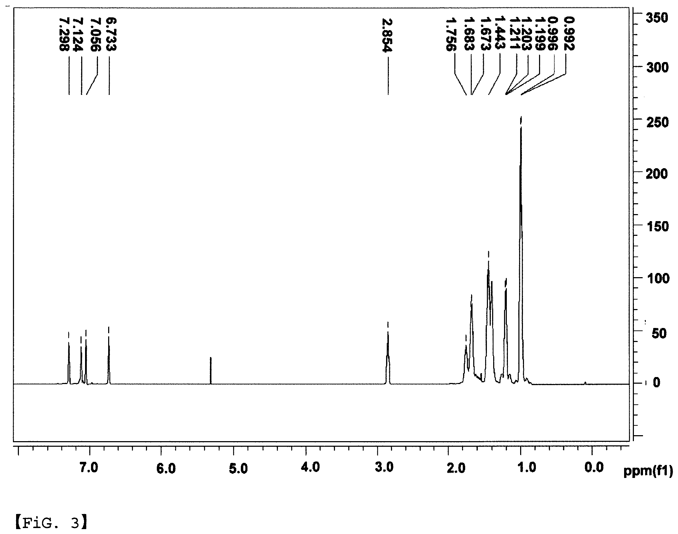

FIG. 2 is a diagram showing an NMR spectrum of the compound obtained in Example 2.

### Example 3. Monomer Synthesis-3'

### (Synthesis of 2,6-Bis(trimethyltin)-4,8-dioctyloxybenzo[1,2-b:4,5-b']dithiophene)

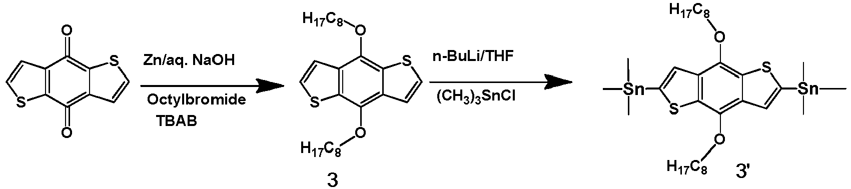

**3.** After 4,8-dehydrobenzo[1,2-b:4,5-b']dithiophene-4,8-dione (4.0 g, 18.1 mmol) and Zn powder (2.6 g, 39.8 mmol) were placed in 60 ml of DI water (H₂O), the mixture was stirred, and sodium hydroxide (NaOH, 12 g) was added thereto, and the result was refluxed for 1 hour while stirring. During the reaction, the color of the solution changed from yellow to orange through red. 1-Bromooctane (10.4 g, 54.3 mmol) and tetrabutylammonium bromide (as catalyst) were added to this solution, and the result was stirred/refluxed for 2 hours. When the color of the solution was red or deep red, zinc powder was additionally added, and the result was stirred/refluxed for 6 hours. This solution was poured into cold water, extracted twice using diethyl ether, and then residual water was removed using magnesium sulfate (MgSO₄). The remaining solution was vacuumed to remove the solvent, and recrystallized twice with ethyl alcohol to obtain a colorless crystalline solid.
Yield: 89.7%

**3'.** 3 (2.0 g, 4.45 mmol) was placed and dissolved in 50 ml of tetrahydrofuran (THF), and the temperature was lowered to -78°C. 1.6 M n-butyllithium (n-BuLi) in hexane (6.12 ml, 9.80 mmol) was slowly added thereto at this temperature, and the result was stirred for 30 minutes. After that, the temperature was raised to 0°C, and the result was stirred for 1 hour under this condition. Then, the temperature was lowered again to - 78°C, and 1 M trimethyltinchloride in tetrahydrofuran (THF) (10.2 ml, 10.25 mmol) was added thereto at once, the temperature was raised to room temperature, and the result was stirred for 12 hours. This solution was poured into ice, extracted twice using diethyl ether, washed twice with water, and residual water was removed using magnesium sulfate (MgSO₄). The remaining solution was vacuumed to remove the solvent, and recrystallized with ethanol to obtain a colorless crystalline solid.
Yield: 57%

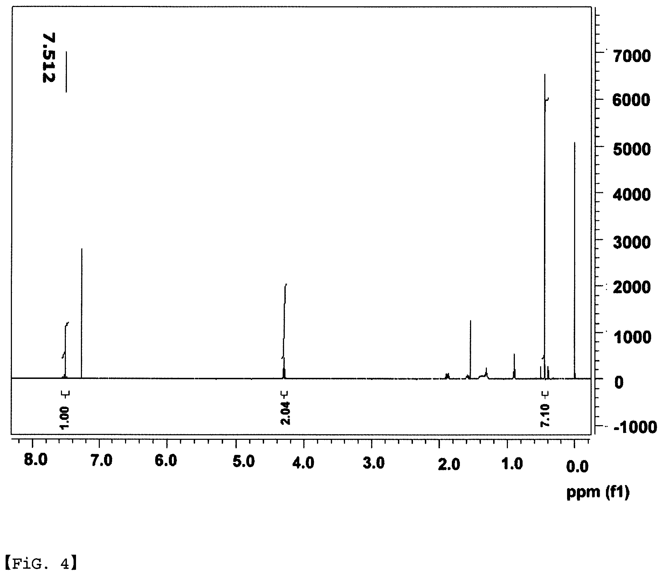

FIG. 3 is a diagram showing an NMR spectrum of the compound obtained in Example 3.

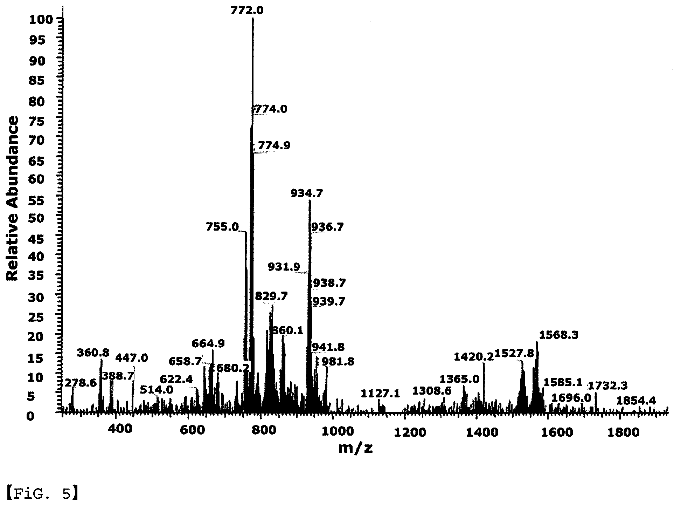

FIG. 4 is a diagram showing an MS spectrum of the compound obtained in Example 3.

### Example 4. Monomer Synthesis-4' (Synthesis of 2,6-Bis(trimethyltin)-4,8-di(2-ethylhexyloxy)benzo[1,2-b:4,5-b']dithiophene)

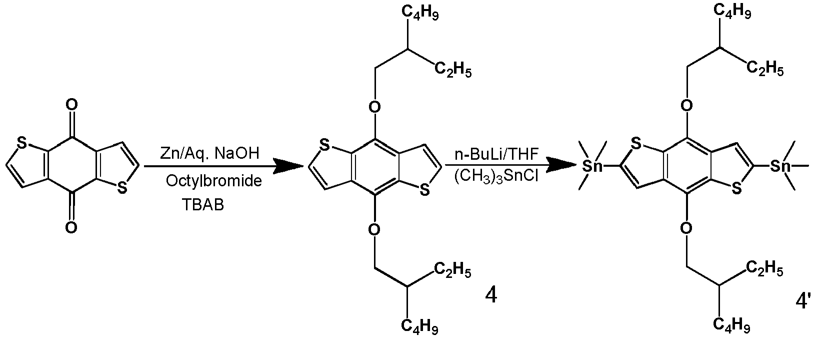

**4.** After 4,8-dehydrobenzo[1,2-b:4,5-b']dithiophene-4,8-dione (8.0 g, 36.2 mmol) and Zn powder (5.2 g, 79.6 mmol) were placed in 60 ml of DI water (H₂O), the mixture was stirred, and sodium hydroxide (NaOH, 24 g) was added thereto, and the result was refluxed for 1 hour while stirring. During the reaction, the color of the solution changed from yellow to orange through red. 2-Ethylhexylbromide (21.0 g, 108.9 mmol) and tetrabutylammonium bromide (as catalyst) were added to this solution, and the result was stirred/refluxed for 2 hours. When the color of the solution was red or deep red, zinc powder was additionally added, and the result was stirred/refluxed for 6 hours. This solution was poured into cold water, extracted twice using diethyl ether, and residual water was removed using magnesium sulfate (MgSO₄). The remaining solution was vacuumed to remove the solvent, and a colorless liquid was obtained through a silica column (eluent; Pet ether:MC=9:1).
Yield: 64.9%

**4'.** 4 (10.3 g, 23.1 mmol) was placed and dissolved in 50 ml of tetrahydrofuran (THF), and the temperature was lowered to -78°C. 1.6 M n-butyllithium (n-BuLi) in hexane (31.7 ml, 50.8 mmol) was slowly added thereto at this temperature, and the result was stirred for 30 minutes. After that, the temperature was raised to 0°C, and the result was stirred for 1 hour under this condition. Then, the temperature was lowered again to - 78°C, and 1 M trimethyltinchloride in tetrahydrofuran (THF) (53.1 ml, 53.1 mmol) was added thereto at once, the temperature was raised to room temperature, and the result was stirred for 12 hours. This solution was poured into ice, extracted twice using diethyl ether, washed twice with water, and residual water was removed using magnesium sulfate (MgSO₄). The remaining solution was vacuumed to remove the solvent, and recrystallized with ethanol to obtain a colorless crystalline solid.
Yield: 71.4%

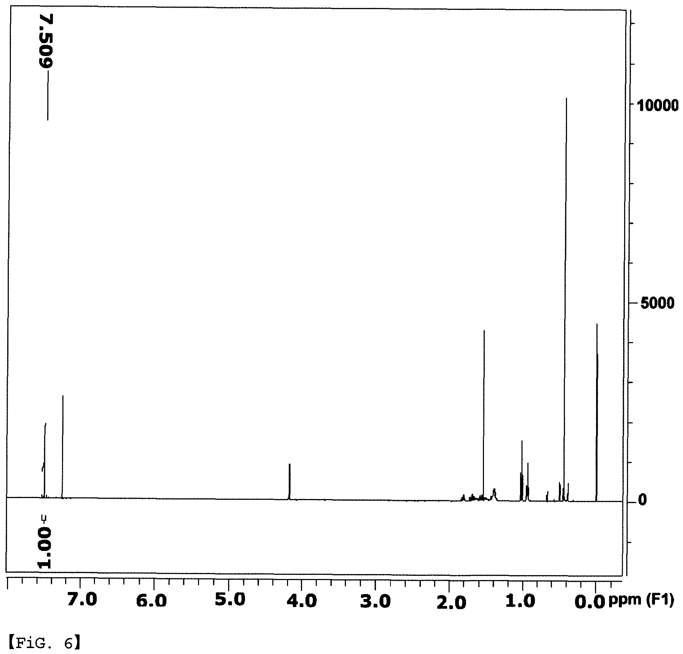

FIG. 5 is a diagram showing an NMR spectrum of the compound obtained in Example 4.

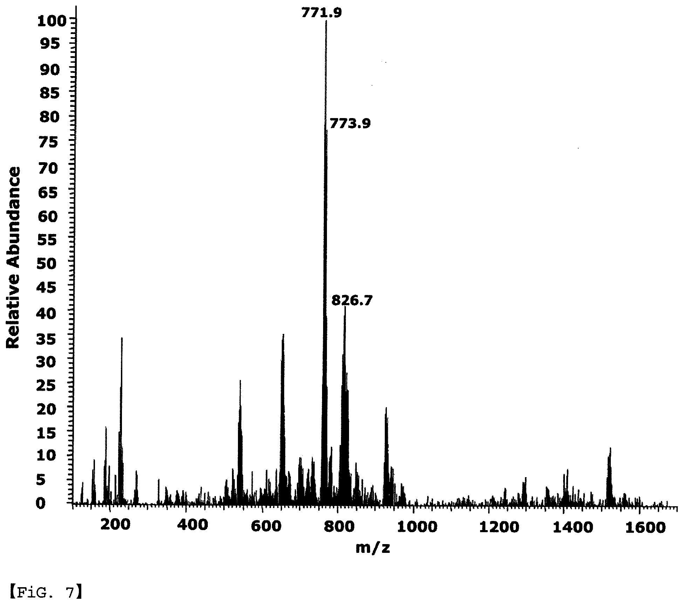

FIG. 6 is a diagram showing an MS spectrum of the compound obtained in Example 4.

### Example 5. Monomer Synthesis-5' (Synthesis of 2,6-Bis(trimethyltin)-4,8-bis(5-(2-hexyl)thiophen-2-yl)benzo[1,2-b:4,5-b']dithiophene)

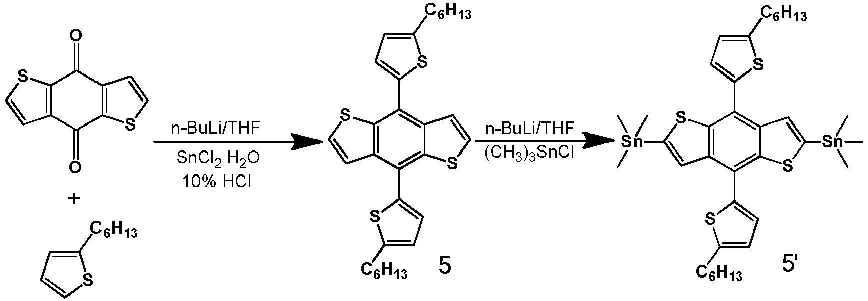

**5.** 2-hexylthiophene (10.0 g, 59.4 mmol) was placed and dissolved in 500 ml of tetrahydrofuran (THF), and the temperature was lowered to -78°C. 2.5 M n-butyllithium (n-BuLi) in hexane (24.0 ml, 59.4 mmol) was slowly added thereto at this temperature, and the result was stirred for 30 minutes. After that, the temperature was raised to 0°C, the result was stirred for 1 hour under this condition, then 4,8-dehydrobenzo[1,2-b:4,5-b']dithiophene-4,8-dione (3.3 g, 14.8 mmol) was added thereto at once, and the result was stirred for 3 hours at 50°C. This solution was cooled to room temperature, tin(II) chloride dehydrate (SnCl_{2.}2H₂O) (26 g) and 10% HCl (56 ml) were added thereto, and the result was further stirred for 3 hours. Ice was poured into this solution, and the result was extracted twice using diethyl ether, washed twice with water, and residual water was removed using magnesium sulfate (MgSO₄). The remaining solution was vacuumed to remove the solvent, and a high-density yellow liquid was obtained through a silica column (eluent; petroleum).
Yield: 64%

**5'.** 5 (3.9 g, 7.59 mmol) was placed and dissolved in 100 ml of tetrahydrofuran (THF), and the temperature was lowered to 0°C. 1.6 M n-butyllithium (n-BuLi) in hexane (10.4 ml, 16.7 mmol) was slowly added thereto at this temperature, and the result was stirred for 1 hour at room temperature. 1 M trimethyltinchloride in THF (22.7 ml, 22.7 mmol) was added to this solution at once, and the result was stirred for 2 hours. Water was poured into this solution, extracted twice using diethyl ether, washed twice with water, and residual water was removed using magnesium sulfate (MgSO₄). The remaining solution was vacuumed to remove the solvent, and recrystallized with ethanol to obtain a light yellow crystalline solid.
Yield: 87%

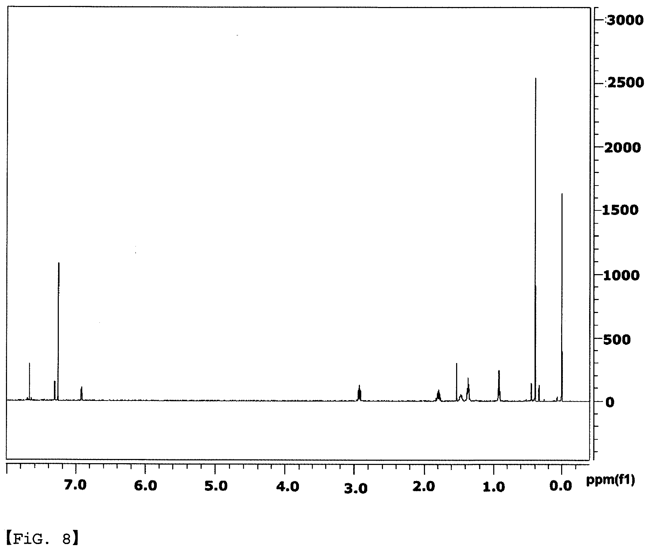

FIG. 7 is a diagram showing an NMR spectrum of the compound obtained in Example 5.

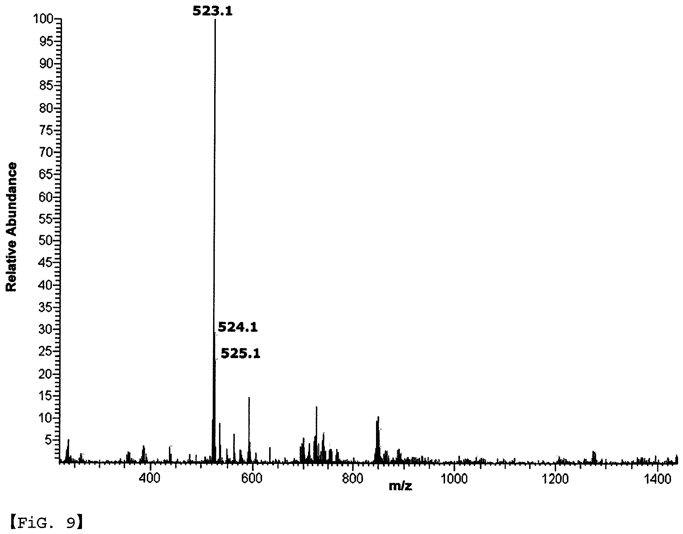

FIG. 8 is a diagram showing an MS spectrum of the compound obtained in Example 5.

### Example 6. Monomer Synthesis-6' (Synthesis of 2,6-Bis(trimethyltin)-4,8-bis(5-(2-ethylhexyl)thiophen-2-yl)benzo[1,2-b:4,5-b']dithiophene)

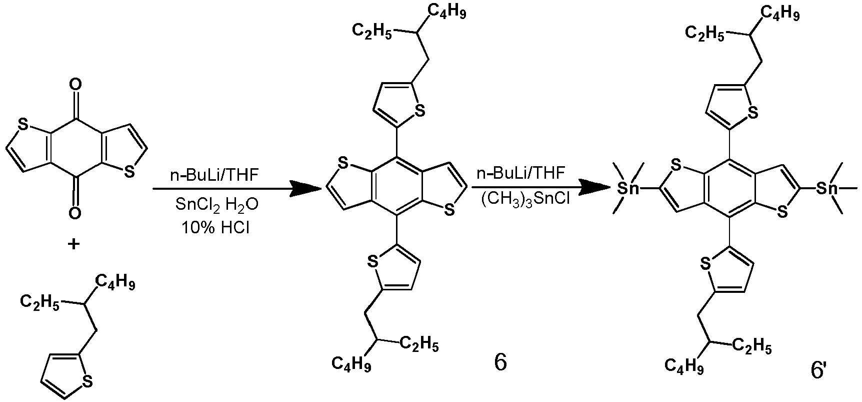

**6.** 2-(2-ethylhexyl)thiophene (10.0 g, 59.4 mmol) was placed and dissolved in 500 ml of tetrahydrofuran (THF), and the temperature was lowered to -78°C. 2.5 M n-butyllithium (n-BuLi) in hexane (24.0 ml, 59.4 mmol) was slowly added thereto at this temperature, and the result was stirred for 30 minutes. After that, the temperature was raised to 0°C, the result was stirred for 1 hour under this condition, then 4,8-dehydrobenzo[1,2-b:4,5-b']dithiophene-4,8-dione (3.3 g, 14.8 mmol) was added thereto at once, and the result was stirred for 3 hours at 50°C. This solution was cooled to room temperature, tin(II) chloride dehydrate (SnCl_{2.}2H₂O) (26 g) and 10% HCl (56 ml) were added thereto, and the result was further stirred for 3 hours. Ice was poured into this solution, and the result was extracted twice using diethyl ether, washed twice with water, and residual water was removed using magnesium sulfate (MgSO₄). The remaining solution was vacuumed to remove the solvent, and a high-density yellow liquid was obtained through a silica column (eluent; petroleum).
Yield: 64%

**6'. 6** (3.9 g, 7.59 mmol) was placed and dissolved in 100 ml of tetrahydrofuran (THF), and the temperature was lowered to 0°C. 1.6 M n-butyllithium (n-BuLi) in hexane (10.4 ml, 16.7 mmol) was slowly added thereto at this temperature, and the result was stirred for 1 hour at room temperature. 1 M trimethyltinchloride in tetrahydrofuran (THF) (22.7 ml, 22.7 mmol) was added to this solution at once, and the result was stirred for 2 hours. Water was poured into this solution, and the result was extracted twice using diethyl ether, washed twice with water, and residual water was removed using magnesium sulfate (MgSO₄). The remaining solution was vacuumed to remove the solvent, and recrystallized with ethanol to obtain a light yellow crystalline solid.
Yield: 87%

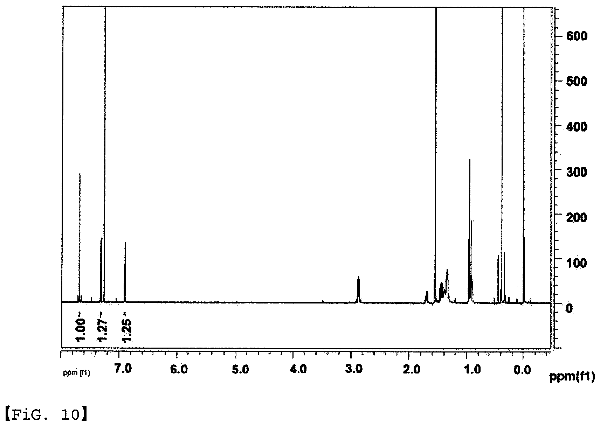

FIG. 9 is a diagram showing an NMR spectrum of the compound obtained in Example 6.

### Example 7. Monomer Synthesis-7 (Synthesis of 2,5-diethylhexyl-3,6-dithiophen-2-ylpyrrolo[3,4-c]pyrrole-1,4-dione)

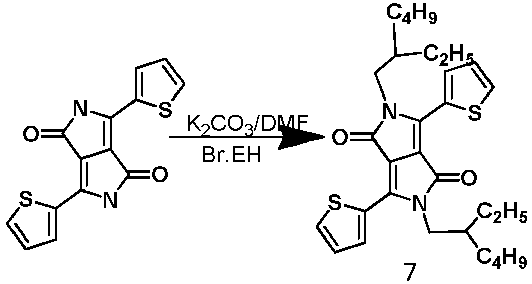

3,6-Dithiophen-2-yl-2,5-dihydropyrrolo[3,4-c]pyrrole-1,4-dione (13.0 g, 43.3 mmol) and potassium carbonate (K₂CO₃, 24.0 g) were placed in 250 ml of dimethylformamide (DMF) and well dissolved by heating at 145°C. 2-Ethylhexylbromide (38.6 g, 200 mmol) was added to this solution at once using a syringe. After the result was stirred for 15 hours or longer at 145°C, the temperature was lowered to room temperature. The result was poured into 500 ml or more of cold water, stirred, and then filtered while washing several times with water and alcohol. After drying, a dark purple solid powder was obtained through a silica column (eluent; hexane:methylene chloride=1:10).
Yield: 87.4%

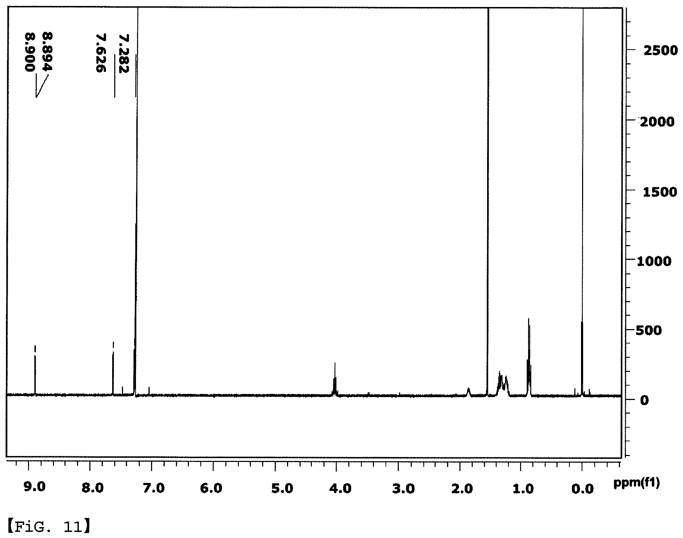

FIG. 10 is a diagram showing an NMR spectrum of 2,5-diethylhexyl-3,6-dithiophen-2-ylpyrrolo[3,4-c]pyrrole-1,4-dione obtained in Example 7.

### Example 8. Monomer Synthesis-8 (Synthesis of 3-(5-bromothiophene-2-yl)-2,5-bis(2-ethylhexyl)-6-(thiophen-2-yl)pyrrolo[3,4-c]pyrrole-1,4-dione)

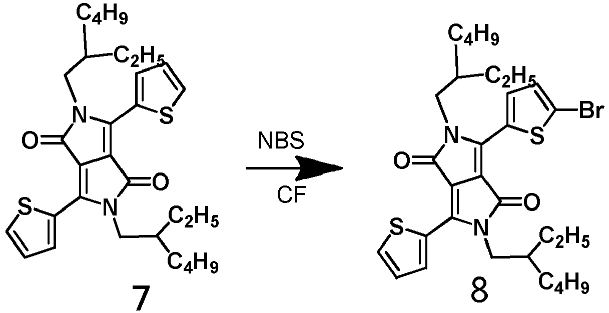

2,5-Diethylhexyl-3,6-dithiophen-2-ylpyrrolo[3,4-c]pyrrole-1,4-dione (5.0 g, 9.25 mmol) and N-bromosuccinimide (NBS, 1.60 g, 9.99 mmol) were placed in 600 ml of chloroform and the mixture was stirred well at room temperature while blocking light. After the reaction was checked with thin-layer chromatography, a dark purple solid powder was obtained through a silica column (eluent; hexane:methylene chloride=1:10).
Yield: 59.7%

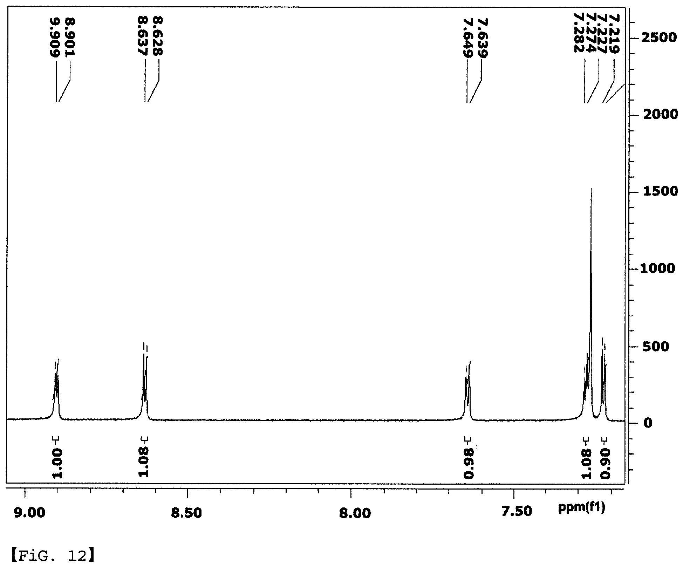

FIG. 11 is a diagram showing an NMR spectrum of the compound prepared in Example 8.

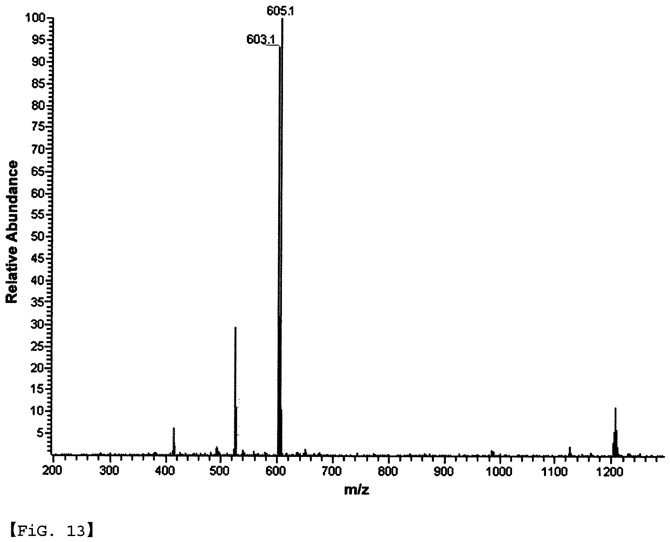

FIG. 12 is a diagram showing an MS spectrum of the compound prepared in Example 8.

### Example 9. Monomer Synthesis 9

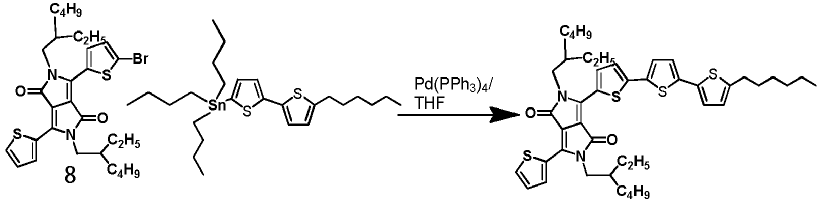

3-(5-bromothiophene-2-yl)-2,5-bis(2-ethylhexyl)-6-(thiophen-2-yl)pyrrolo[3,4-c]pyrrole-1,4-dione (8.0 g, 13.2 mmol), 5-tri-n-butylstannyl)-5'-hexyl-2,2'-bithiophene (8.57 g, 15.9 mmol) and tetrakis(triphenylphosphine)palladium(0) (Pd(PPh₃)₄, 0.46 g) were placed and dissolved in 300 ml of tetrahydrofuran (THF). This solution was reacted for 24 hours while being stirred/refluxed. This solution was poured into water, extracted twice using methylene chloride, washed twice with water, and residual water was removed using magnesium sulfate. The remaining solution was vacuumed to remove the solvent, and a fine khaki powder was obtained through a silica column (eluent; hexane/MC=10:2).
Yield: 57%

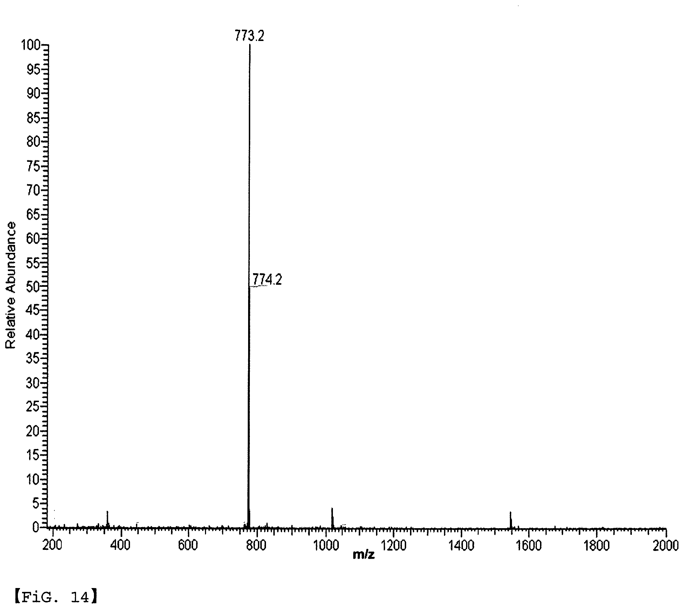

FIG. 13 is a diagram showing an MS spectrum of the compound obtained in Example 9.

### Example 10. Monomer Synthesis-10

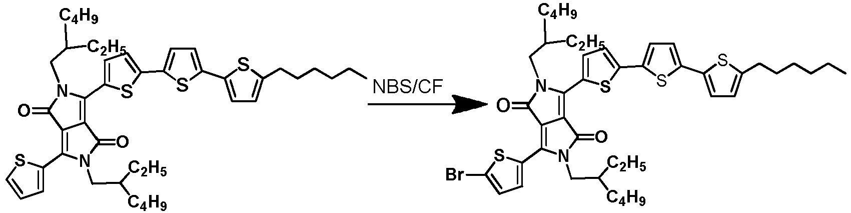

Monomer 9 (3.0 g, 3.88 mmol) and N-bromosuccinimide (NBS, 0.76 g, 4.26 mmol) were placed in 500 ml of chloroform, and the mixture was stirred at room temperature while blocking light. After the reaction was checked with thin-layer chromatography, a dark purple solid powder was obtained through a silica column (eluent; hexane/MC=10:2).
Yield: 87%

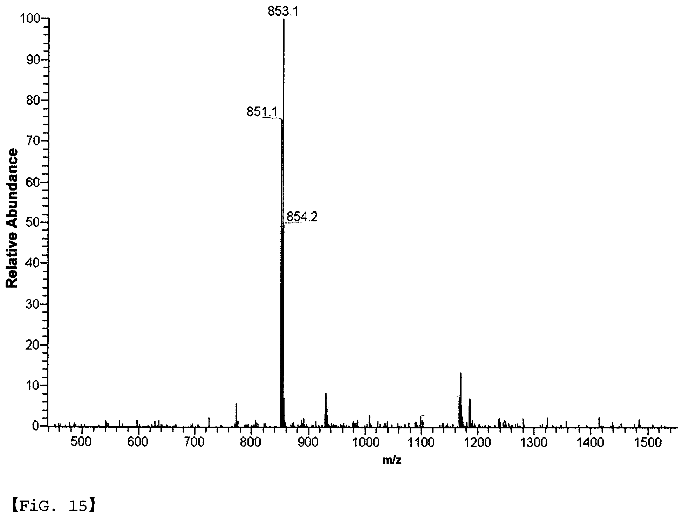

FIG. 14 is a diagram showing an MS spectrum of the compound obtained in Example 10.

### Example 11. Monomer Synthesis-11

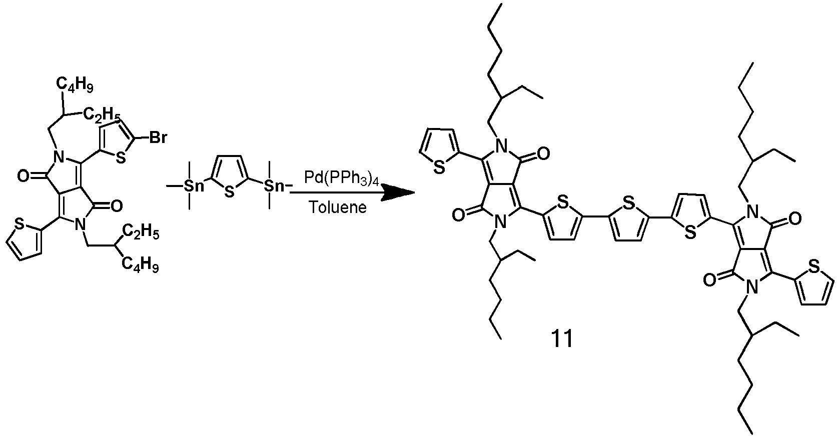

2,5-Bis(trimethylstannyl)thiophene (1.5 g, 3.66 mmol), 3-(5-bromothiophene-2-yl)-2,5-bis(2-ethylhexyl)-6-(thiophen-2-yl)pyrrolo[3,4-c]pyrrole-1,4-dione (4.64 g, 7.68 mmol) and tetrakis(triphenylphosphine)palladium(0) (Pd(PPh₃)₄, 0.27 g) were placed and dissolved in 120 ml of toluene. This solution was reacted for 24 hours while being stirred/refluxed. This solution was poured into water, extracted twice using methylene chloride, washed twice with water, and residual water was removed using magnesium sulfate. The remaining solution was vacuumed to remove the solvent, and a fine powder was obtained through a silica column (eluent; hexane/MC=2:1).
Yield : 72%

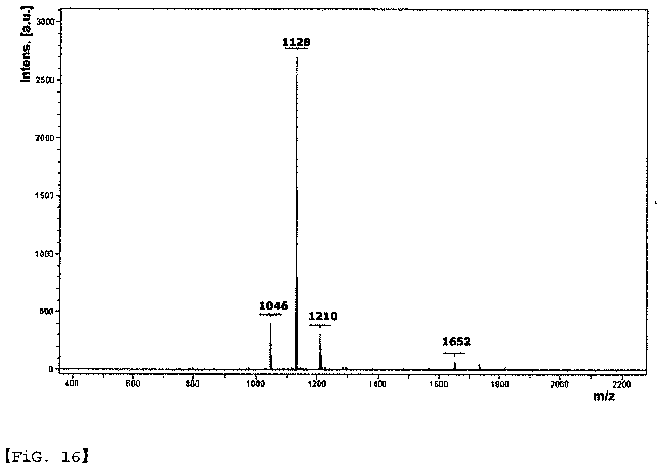

FIG. 15 is a diagram showing an MS spectrum of the compound obtained in Example 11.

### Example 12. Monomer Synthesis-12

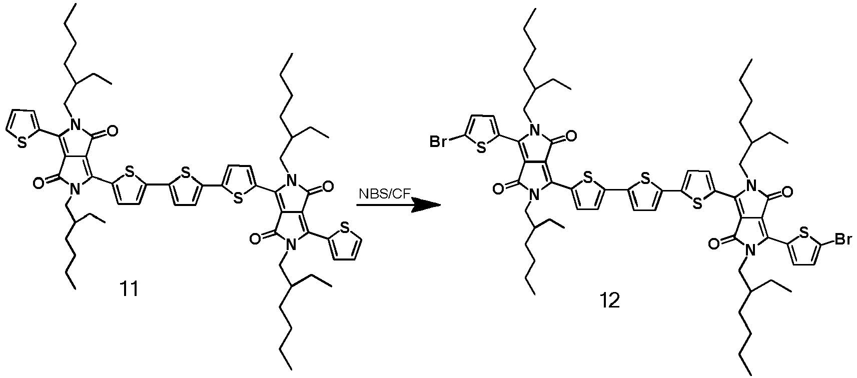

Compound 11 (1.0 g, 0.88 mmol) and N-bromosuccinimide (NBS, 0.34 g, 1.93 mmol) were placed in 300 ml of chloroform, and the mixture was stirred at room temperature while blocking light. After the reaction was checked with thin-layer chromatography, a dark purple solid powder was obtained through a silica column (eluent; hexane/MC=4:1).
Yield: 64%

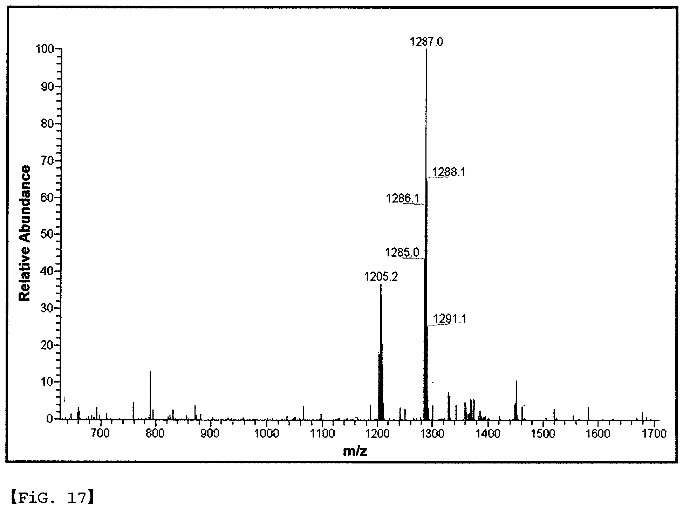

FIG. 16 is a diagram showing an MS spectrum of the compound obtained in Example 12.

### Example 13. Monomer Synthesis-13

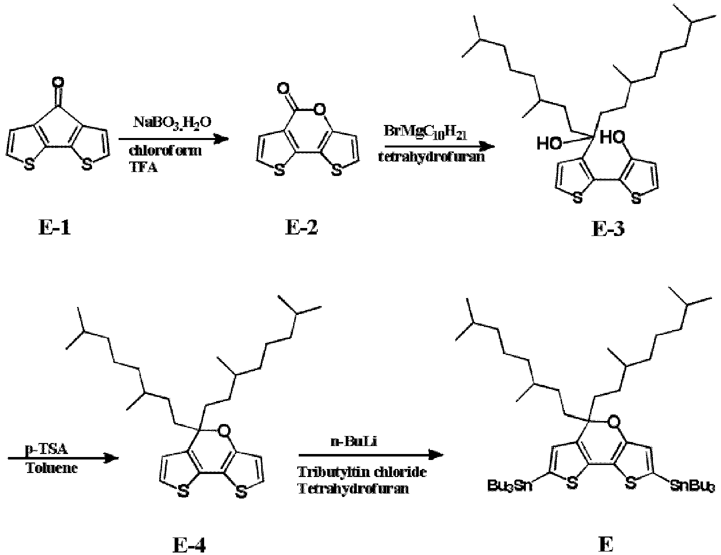

### (1) Synthesis of Chemical Formula E-2

Compound E-1 (6.0 g, 31.2 mmol) was dissolved in 50 ml chloroform (CF):50 ml trifluoroacetic acid (TFA). Sodiumperborate monohydrate (7.39 g, 72.8 mmol) was added thereto at once, and the result was stirred for 1 hour at room temperature. This solution was poured into water, and then extracted using chloroform. The solvent was removed under vacuum, and a white solid was obtained through a silica column (eluent; Hx/MC=1/1).
Yield: 35%

### (2) Synthesis of Chemical Formula E-3

Compound E-2 (2.4 g, 11.4 mmol) was dissolved in 60 ml of tetrahydrofuran (THF) under nitrogen. 25.4 ml of 3,7-dimethyloctylmagnesium bromide (1 M solution in diethyl ether) was slowly injected thereto at -25°C. The result was stirred for 10 hour while raising the temperature to room temperature, and the reaction was stopped while adding 50 ml of water. The result was extracted using ethyl acetate (EA), and residual water was removed using magnesium sulfate (MgSO₄). A light yellow liquid was obtained through a silica column.
Yield: 93%

### (3) Synthesis of Chemical Formula E-4

Compound E-3 (4.5 g, 12.0 mmol) was dissolved in 100 ml of toluene under nitrogen. 300 mg of sodium p-toluenesulfonic acid monohydrate was added thereto, and the result was reacted for 3 hours at 120°C.

The reaction solution was poured into water, and was extracted by adding toluene thereto. The result was dried using magnesium sulfate (MgSO₄), and then the solvent was removed under vacuum. A yellow liquid was obtained through a silica column (eluent; Hx).
Yield: 95%

### (4) Synthesis of Chemical Formula E

Compound E-4 (0.58 g, 1.2 mmol) was dissolved in 20 ml of tetrahydrofuran (THF) under nitrogen. n-Butyl lithium (1.7 ml, 1.6 M solution in hexane) was slowly added thereto at -78°C, the result was stirred for 30 minutes, and then stirred for 2 hours at room temperature. The temperature was lowered to -78°C again, and 0.92 ml of a tributyltin chloride solution was added thereto. The temperature was raised to room temperature, and the result was stirred for 10 hours. The result was poured into water, and extracted using hexane. The solvent was removed under vacuum, and a brown liquid was obtained through a silica column (eluent. Hx, 10% triethylamine).
Yield: 97%

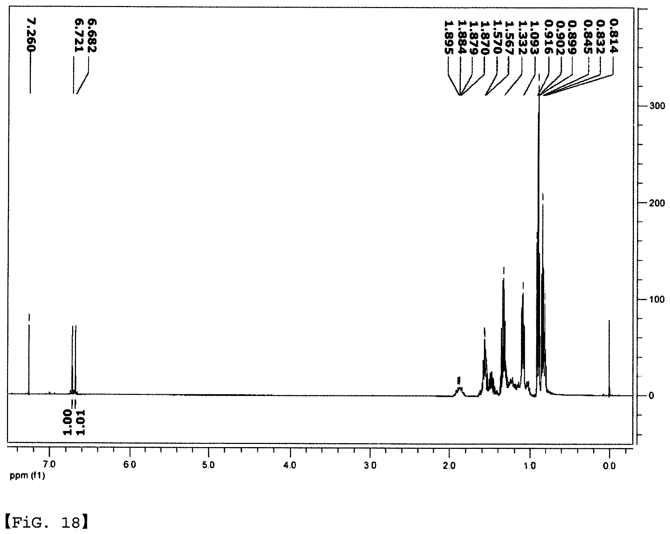

FIG. 17 is a diagram showing an NMR spectrum of Compound E obtained in Example 13.

### Example 14. Synthesis of Single molecule 1 (not in accordance with the invention)

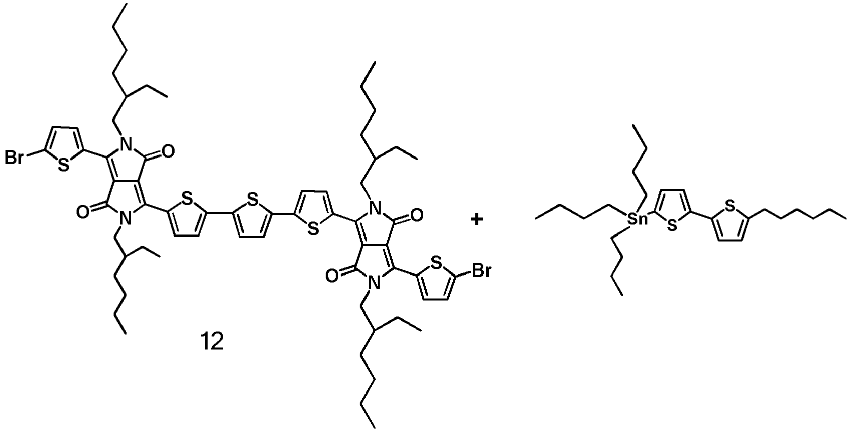

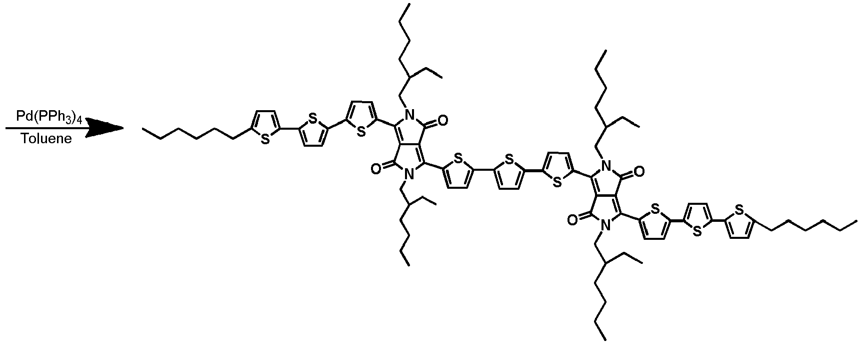

Compound 12 (0.73 g, 0.56 mmol), 5-(tri-n-butylstannyl)-5'-hexyl-2,2'-bithiophene (0.90 g, 1.40 mmol), tetrakis(triphenylphosphine)palladium(0) (Pd(PPh₃)₄, 10 mg) and tri-(o-tolyl)phosphine (20 mg) were placed and dissolved in 30 ml of chlorobenzene. This solution was reacted for 24 hours at 130°C. This solution was poured into water, extracted twice using chloroform, washed twice with water, and residual water was removed using magnesium sulfate. The remaining solution was vacuumed to remove the solvent, and a fine powder was obtained through a silica column (eluent; hexane/MC=10:4). Yield: 63%

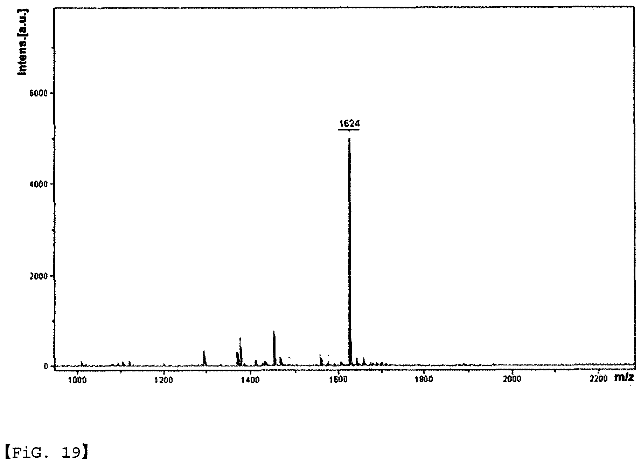

FIG. 18 is a diagram showing an MS spectrum of Single molecule 1 prepared in Example 14.

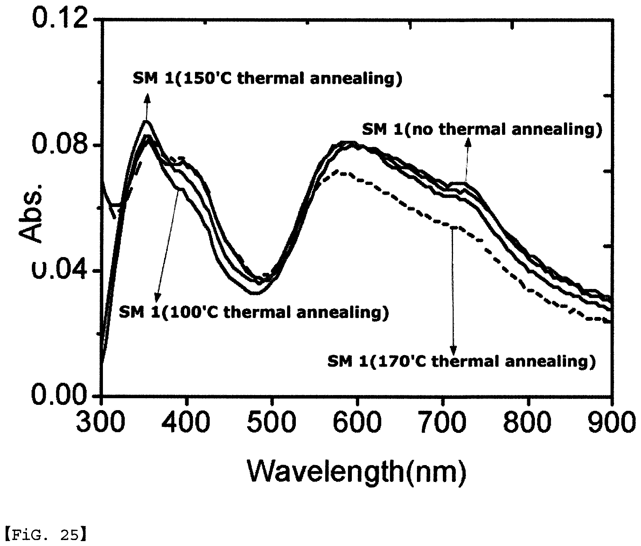

FIG. 24 is a diagram showing a UV-Vis absorption spectrum in a film phase in which the chlorobenzene solution of Single molecule 1 of Example 14 was heat treated.

The film-phase UV absorption spectrum of FIG. 24 was analyzed using a UV-Vis absorption spectrometer after dissolving the compound in chlorobenzene to have a concentration of 1 wt%, dropping this solution on a glass substrate, spin coating the sample for 60 seconds at 1000 rpm, and heat treating the result at 25°C, 100°C, 150°C and 170°C.

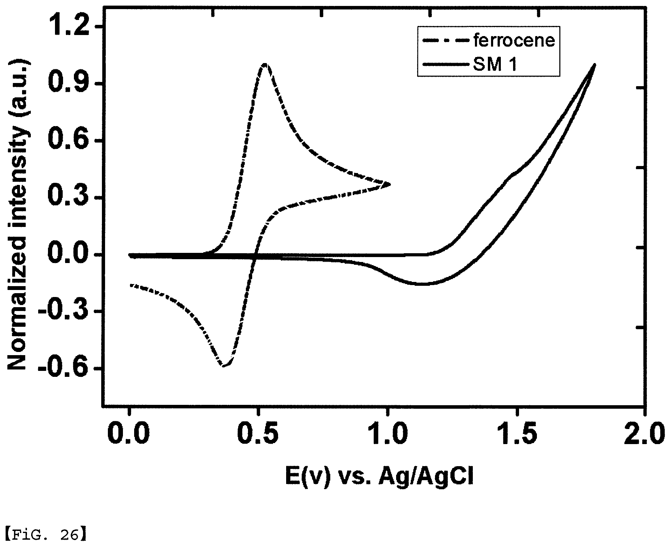

FIG. 25 is a diagram showing an electrochemical measurement result (cyclic voltametry) of Single molecule 1 prepared in Example 14.

The electrochemical measurement (cyclic voltametry) of FIG. 25 was analyzed using a three-electrode system in which a glassy carbon working electrode, an Ag/Agcl reference electrode, and a Pt electrode were put in an electrolyte solution dissolving Bu₄NBF₄ in acetonitrile to have a concentration of 0.1 M. The compound was coated on the working electrode using a drop casting method.

### Example 15. Synthesis of Single molecule 2 (not in accordance with the invention)

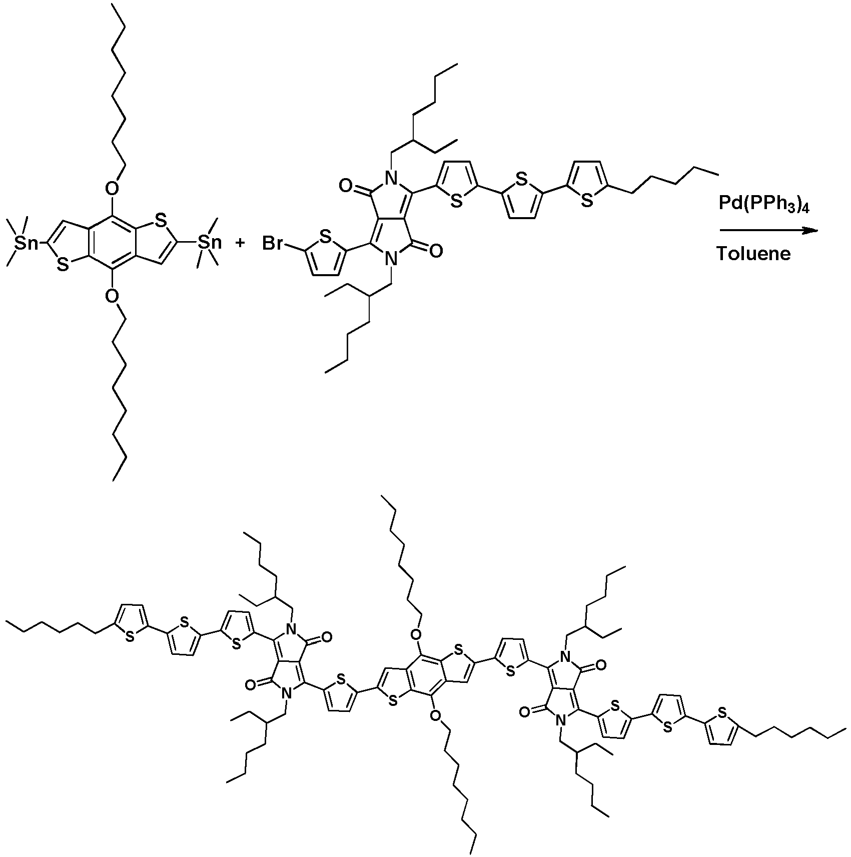

2,6-Bis(trimethyltin)-4,8-dioctyloxybenzo[1,2-b:4,5-b']dithiophene (0.425 g, 0.50 mmol), Compound 10 (0.95 g, 1.11 mmol) and tetrakis(triphenylphosphine)palladium(0) (Pd(PPh₃)₄, 40 mg) were placed in 100 ml of toluene. This solution was reacted for 24 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:4).
Yield: 52%

### Example 16. Synthesis of Single molecule 3 (not in accordance with the invention)

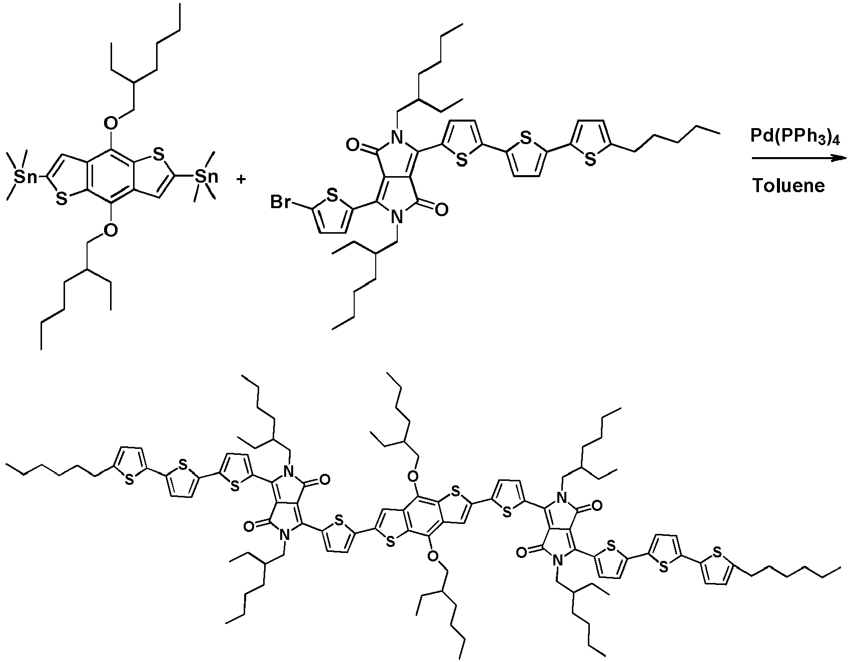

2,6-Bis(trimethyltin)-4,8-di(2-ethylhexyloxy)benzo[1,2-b:4,5-b']dithiophene (0.425 g, 0.41 mmol), Compound 10 (1.00 g, 1.17 mmol), and tetrakis(triphenylphosphine)palladium(0) (Pd(PPh₃)₄, 40 mg) were placed in 100 ml of toluene. This solution was reacted for 24 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:4).
Yield: 50%

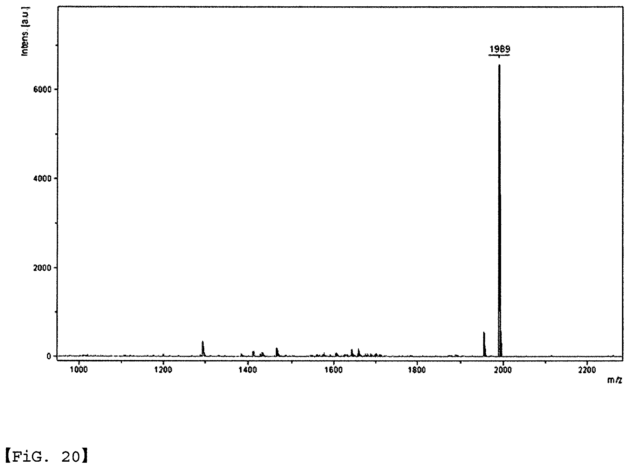

FIG. 19 is a diagram showing an MS spectrum of Single molecule 3 prepared in Example 16.

### Example 17. Synthesis of Single molecule 4

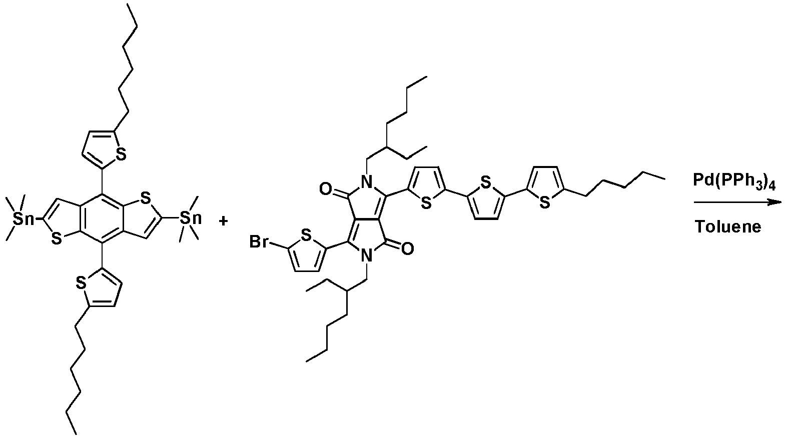

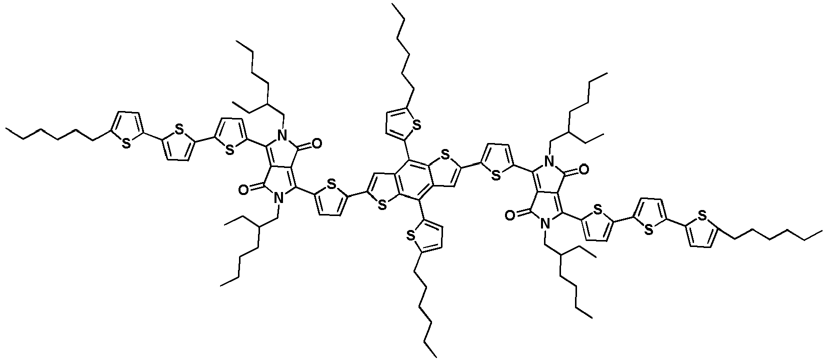

2,6-Bis(trimethyltin)-4,8-bis(5-(2-hexyl)thiophen-2-yl)benzo[1,2-b:4,5-b']dithiophene (0.425 g, 0.41 mmol), Compound 10 (0.95 g, 1.11 mmol) and tetrakis(triphenylphosphine)palladium(0) (Pd(PPh₃)₄, 40 mg) were placed in 100 ml of toluene. This solution was reacted for 24 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:4) .

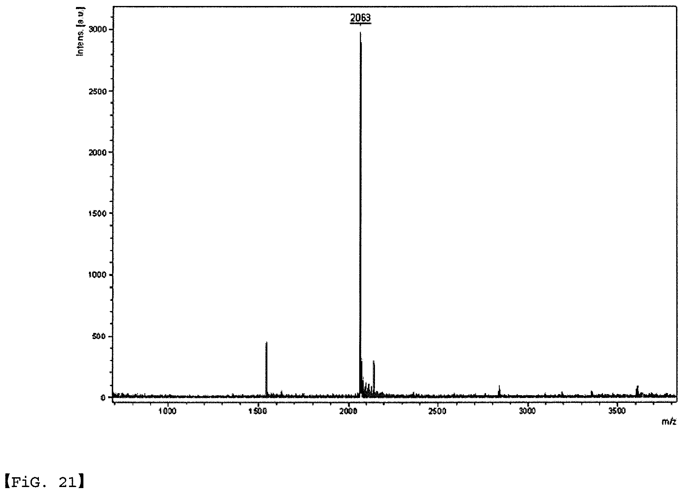

FIG. 20 is a diagram showing an MS spectrum of Single molecule 4 prepared in Example 17.

### Example 18. Synthesis of Single molecule 5

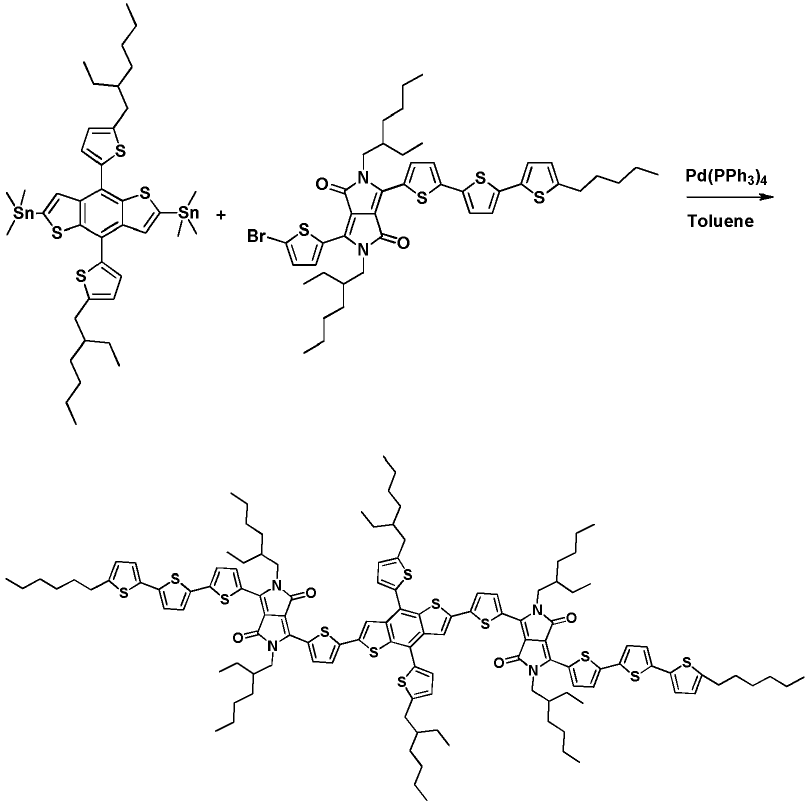

2,6-Bis(trimethyltin)-4,8-bis(5-(2-ethylhexyl)thiophen-2-yl)benzo[1,2-b:4,5-b']dithiophene (0.53 g, 0.58 mmol), Compound 10 (1.00 g, 1.17 mmol) and tetrakis(triphenylphosphine)palladium(0) (Pd(PPh₃)₄, 40 mg) were placed in 100 ml of toluene. This solution was reacted for 24 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:4) .
Yield: 43%

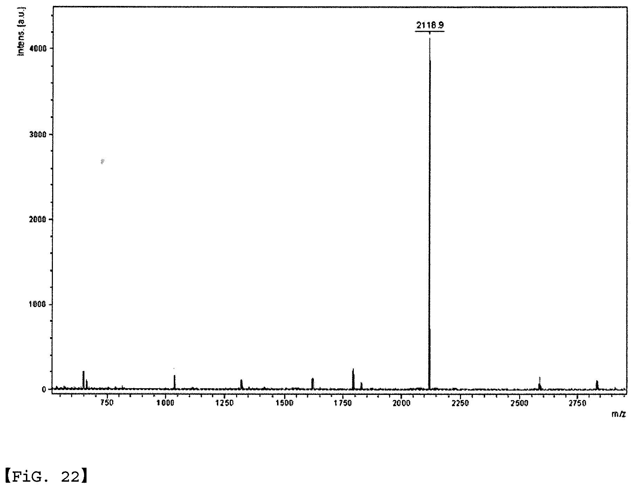

FIG. 21 is a diagram showing an MS spectrum of Single molecule 5 prepared in Example 18.

### Example 19. Synthesis of Single molecule 6

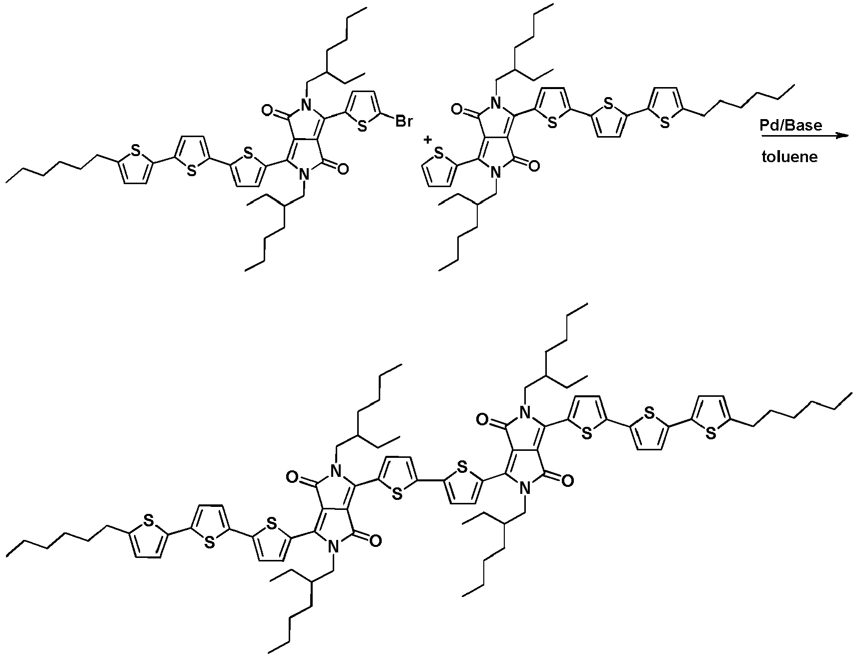

Material 9 (0.27 g, 0.35 mmol), material 10 (0.30 g, 0.35 mmol) and tetrakis(triphenylphosphine)palladium(0) (Pd(PPh₃)₄, 10 mg) were placed in 50 ml of toluene. This solution was reacted for 48 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:5).

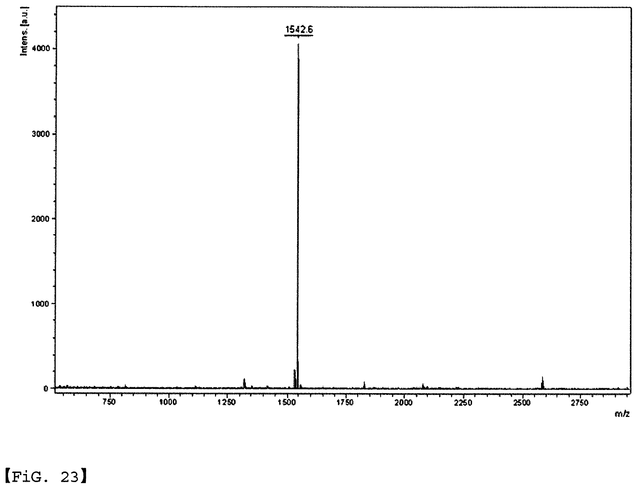

FIG. 22 is a diagram showing an MS spectrum of Single molecule 6 prepared in Example 19.

### Example 20. Synthesis of Single molecule 7

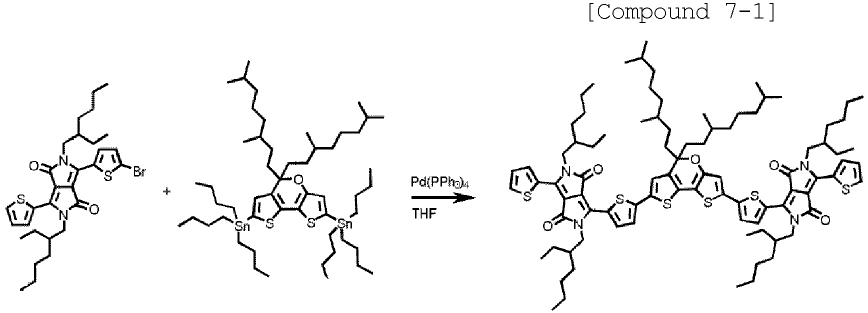

5,5-Bis(3,7-dimethyloctyl)-5H-dithieno[3,2-b:2' ,3'-d]pyran-2,7-diyl)bis(tristannane) (2.0 g, 1.8996 mmol), 3-(5-Bromothiophen-2-yl)-2,5-bis(2-ethylhexyl)-6-(thiophen-2-yl)pyrrolo[3,4-c]pyrrole-1,4(2H,5H)-dione (2.30 g, 3.7991 mmol) and tetrakis(triphenylphosphine)palladium(0) (Pd(PPh₃)₄, 0.13 g) were placed and dissolved in 300 ml of tetrahydrofuran (THF). This solution was reacted for 20 hours at 100°C. This solution was poured into water, extracted twice using methylene chloride, washed twice with water, and residual water was removed using magnesium sulfate. The remaining solution was vacuumed to remove the solvent, and a brown solid was obtained through a silica column (eluent; hexane/MC=10:2).
Yield: 78%

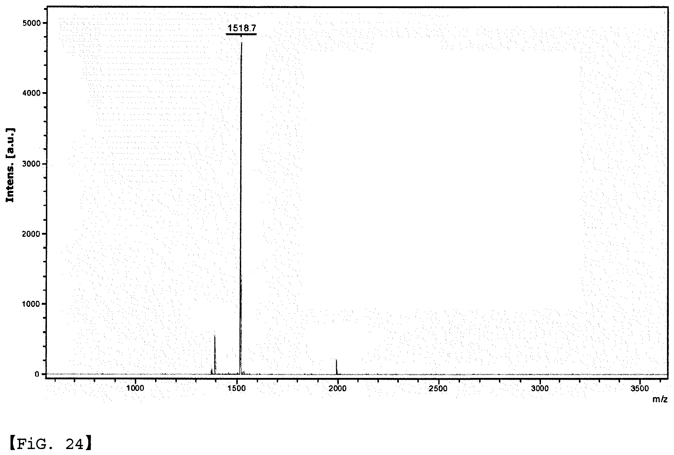

FIG. 23 is a diagram showing an MS spectrum of Compound 7-1 prepared in Example 20.

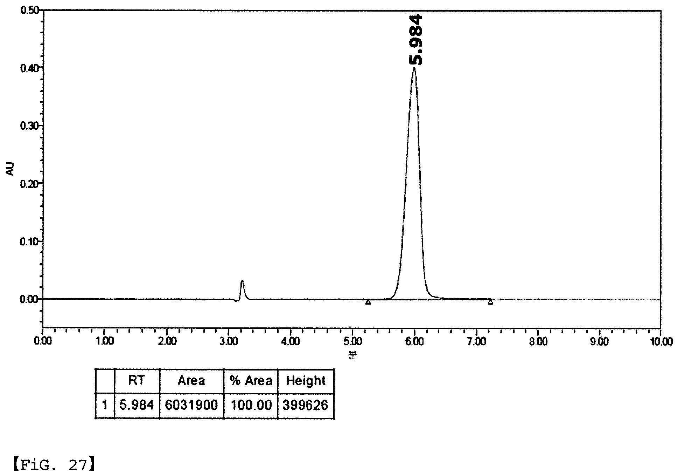

FIG. 26 is a diagram showing high performance liquid chromatography (HPLC) of Compound 7-1 prepared in Example 20.

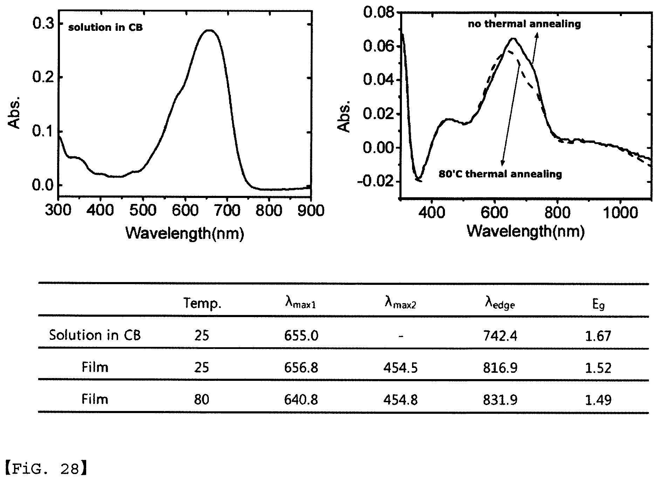

FIG. 27 shows a UV-Vis absorption spectrum of Compound 7-1 of Example 20 in a chlorobenzene solution and in a film phase in which the chlorobenzene solution is heat treated.

The UV absorption spectrum of FIG. 27 was was analyzed using a UV-Vis absorption spectrometer of 1) the absorption spectrum of a sample dissolving the compound in chlorobenzene to have a concentration of 1 wt%, and 2) the film-phase UV absorption spectrum after dissolving the compound in chlorobenzene to have a concentration of 1 wt%, dropping this solution on a glass substrate, spin coating the sample for 60 seconds at 1000 rpm, and heat treating the result at 80°C.

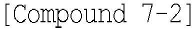

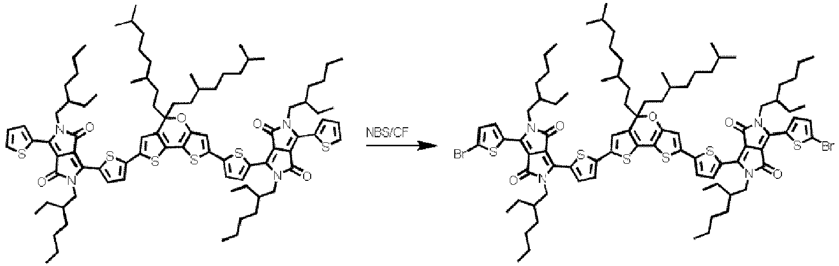

M1 (0.720 g, 0.4736 mmol) was well dissolved in 100 ml of chloroform (CF). N-bromosuccinimide (NBS) (0.17 g, 0.9613 mmol) was added to this solution and was stirred. The result was extracted using distilled water and ethyl acetate (EA), and then moisture in the organic layer was removed using anhydrous sodium sulfate, and solid impurities were removed by vacuum filtration. A fine powder was obtained by separating the result using column chromatography (eluent; MC/Hx=10:5).
Yield: 50.4%

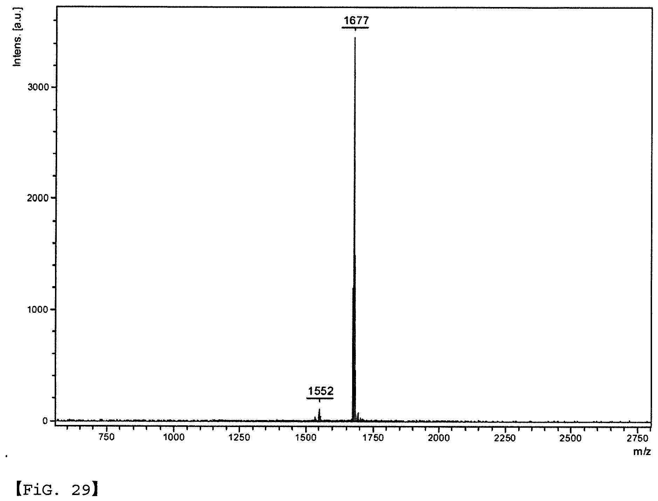

FIG. 28 is a diagram showing an MS spectrum of Compound 7-2 obtained in Example 20.

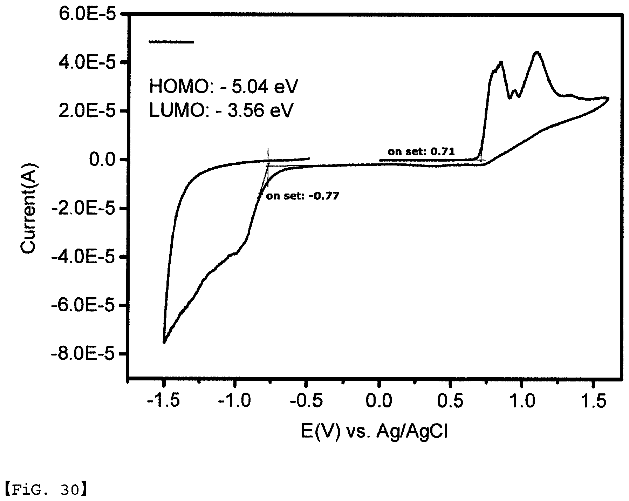

FIG. 29 is a diagram showing an electrochemical measurement result (cyclic voltametry) of Compound 7-2 obtained in Example 20.

The electrochemical measurement (cyclic voltametry) of FIG. 29 was analyzed using a three-electrode system in which a glassy carbon working electrode, an Ag/Agcl reference electrode, and a Pt electrode were put in an electrolyte solution dissolving Bu₄NBF₄ in acetonitrile to have a concentration of 0.1 M. The compound was coated on the working electrode using a drop casting method.

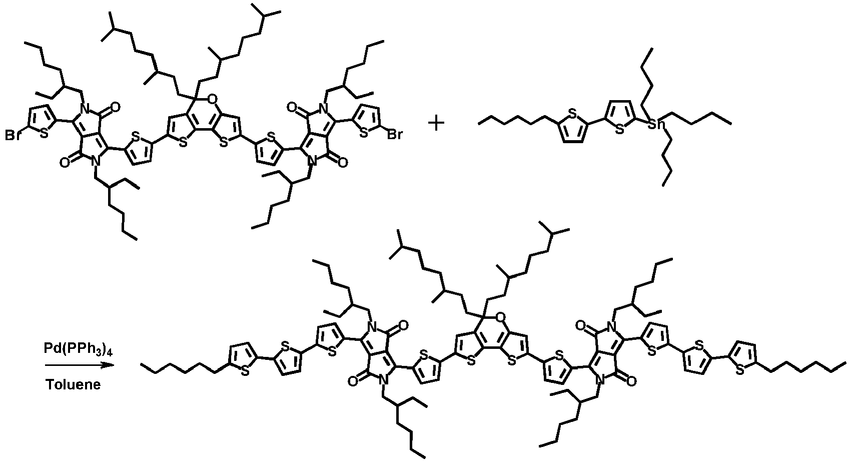

Compound 7-2 (0.50 g, 0.2980 mmol), Monomer 2 (0.40 g, 0.7449 mmol) and tetrakis(triphenylphosphine)palladium(0) (Pd(PPh₃)₄, 30 mg) were placed in 100 ml of toluene. This solution was reacted for 24 hours at 100°C. This solution was poured into water, extracted twice using chloroform, washed twice with water, and residual water was removed using magnesium sulfate (MgSO₄). The remaining solution was vacuumed to remove the solvent, and a fine powder was obtained through a silica column (eluent; hexane/MC=10:4).
Yield: 45%

### Fabrication of Organic Solar Cell and Measurement of Its Properties

### Preparation Example 1. Fabrication of Organic Solar Cell-1 (not in accordance with the invention)

A composite solution was prepared by dissolving Single molecule 1 prepared in Example 14 and PCBM in chlorobenzene (CB) in a ratio of 60:40 (w/w ratio). Herein, the concentration was adjusted to 2.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated

### Preparation Example 2. Fabrication of Organic Solar Cell-2 (not in accordance with the invention)

A composite solution was prepared by dissolving single molecule 2 prepared in Example 15 and PCBM in chlorobenzene (CB) in a ratio of 60:40 (w/w ratio) . Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Preparation Example 3. Fabrication of Organic Solar Cell-3 (not in accordance with the invention)

A composite solution was prepared by dissolving Single molecule 3 prepared in Example 16 and PCBM in chlorobenzene (CB) in a ratio of 60:40 (w/w ratio) . Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Preparation Example 4. Fabrication of Organic Solar Cell-4

A composite solution was prepared by dissolving Single molecule 4 prepared in Example 17 and PCBM in chlorobenzene (CB) in a ratio of 60:40 (w/w ratio). Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Preparation Example 5. Fabrication of Organic Solar Cell-5

A composite solution was prepared by dissolving single molecule 5 prepared in Example 18 and PCBM in chlorobenzene (CB) in a ratio of 60:40 (w/w ratio). Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Preparation Example 6. Fabrication of Organic Solar Cell-6

A composite solution was prepared by dissolving single molecule 6 prepared in Example 19 and PCBM in chlorobenzene (CB) in a ratio of 60:40 (w/w ratio). Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Preparation Example 7. Fabrication of Organic Solar Cell-7

A composite solution was prepared by dissolving single molecule 7 prepared in Example 20 and PCBM in chlorobenzene (CB) in a ratio of 60:40 (w/w ratio). Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

### Comparative Example 1

A composite solution was prepared by dissolving P3HT and PC₆₁BM in chlorobenzene (CB) in a ratio of 1:1 (w/w ratio) . Herein, the concentration was adjusted to 3.0 wt%, and the organic solar cell employed an ITO/PEDOT:PSS/photoactive layer/Al structure. The glass substrate coated with ITO was ultrasonic cleaned using distilled water, acetone and 2-propanol, and after the ITO surface was ozone treated for 10 minutes, the surface was spin-coated with PEDOT:PSS (baytrom P) to a thickness of 45 nm, and then heat treated for 10 minutes at 120°C. In order to coat the photoactive layer, the compound-PCBM composite solution was filtered using a PP syringe filter of 0.45 µm, then spin-coated, and deposited with Al to a thickness of 200 nm using a thermal evaporator under a vacuum of 3x10⁻⁸ torr, and as a result, the organic solar cell was fabricated.

The photoelectric conversion properties of the organic solar cells prepared in Preparation Examples 1 to 6 and Comparative Example 1 were measured under a condition of 100 mW/cm² (AM 1.5), and the results are shown in the following Table 1.

**[Table 1]**

| | Active Layer | V_{OC} (V) | J_{SC} (mA/cm²) | FF | PCE (%) |
|---|---|---|---|---|---|
| Preparation Example 1* | Single molecule 1/PC₆₁BM=60: 40 | 0.82 | 12.2 | 59.2 | 5.93 |
| Preparation Example 2* | Single molecule 2/PC₆₁BM=60:40 | 0.82 | 10.6 | 65.7 | 5.78 |
| Preparation Example 3* | Single molecule 3/PC₆₁BM=60:40 | 0.83 | 12.2 | 65.7 | 6.55 |
| Preparation Example 4 | Single molecule 4/PC₆₁BM=60:40 | 0.84 | 11.6 | 70.3 | 6.90 |
| Preparation | Single molecule | 0.82 | 13.1 | 67.7 | 7.27 |
| Example 5 | 5/PC₆₁BM=60:40 | | | | |
| Preparation Example 6 | Single molecule 6/PC₆₁PM=60:40 | 0.66 | 12.9 | 59.7 | 5.05 |
| Preparation Example 7 | Single molecule 7/PC₆₁BM=60:40 | 0.81 | 11.02 | 58.3 | 5.20 |
| Comparative Example 1 | P3LT/PC₆₁PM=1:1 | 0.62 | 9.97 | 45.5 | 2.81 |

| | | | | | |
|---|---|---|---|---|---|
| *not in accordance with the invention | | | | | |

In Table 1, V_{OC} means an open voltage, J_{SC} means a short-circuit current, FF means a fill factor, and PCE means energy conversion efficiency. The open voltage and the short-circuit current are each an x-axis and a y-axis intercept in the four quadrants of a voltage-current density curve, and as these two values increase, solar cell efficiency is preferably enhanced. In addition, the fill factor is a value dividing the rectangle area that may be drawn inside the curve by the product of the short-circuit current and the open voltage. The energy conversion efficiency may be obtained when these three values are divided by the irradiated light, and it is preferable as the value is higher.

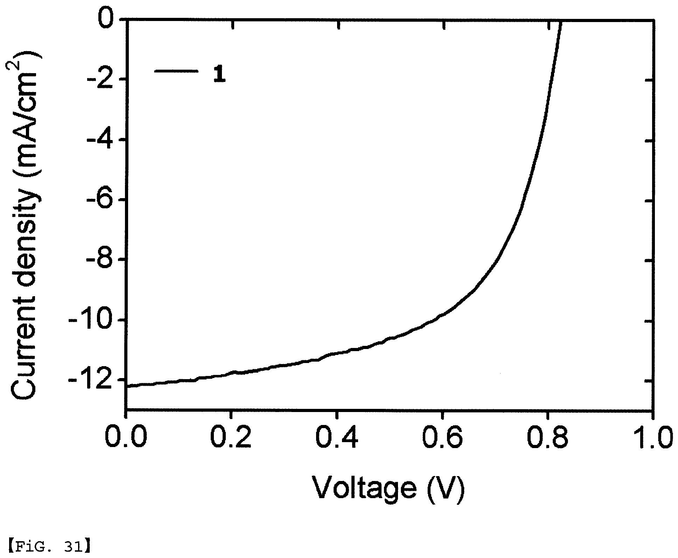

FIG. 30 is a diagram showing the current density by voltage of the organic solar cell including Single molecule 1 prepared in Example 14.

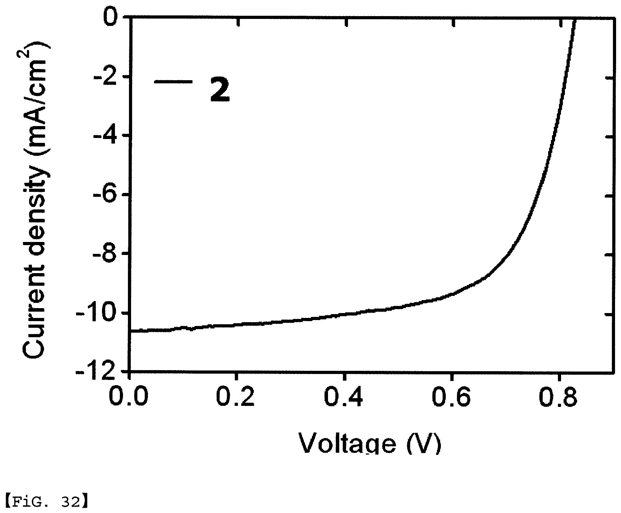

FIG. 31 is a diagram showing the current density by voltage of the organic solar cell including Single molecule 2 prepared in Example 15.

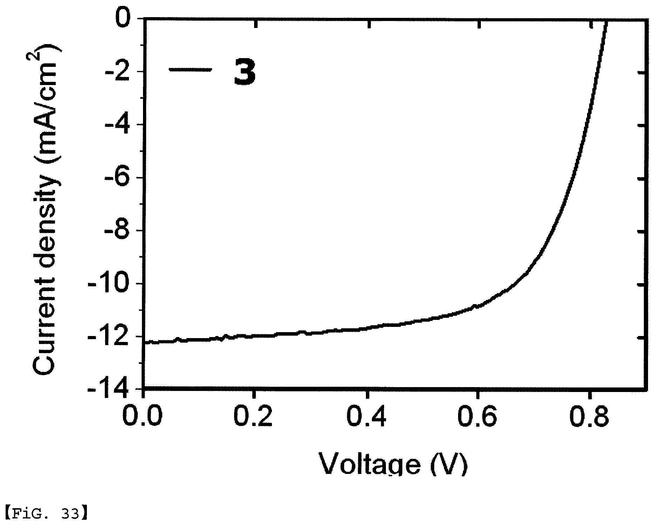

FIG. 32 is a diagram showing the current density by voltage of the organic solar cell including Single molecule 3 prepared in Example 16.

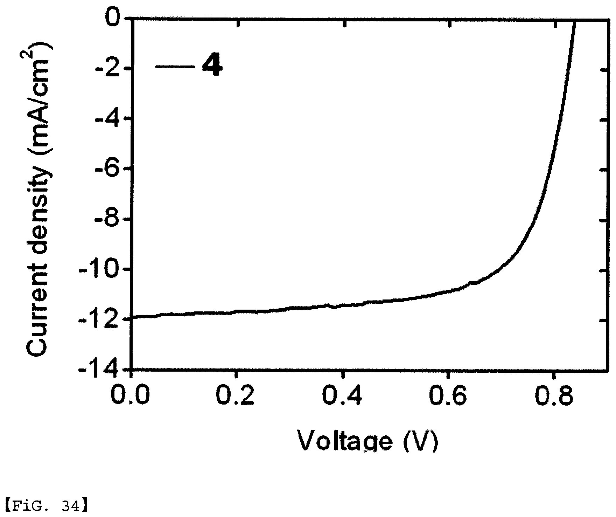

FIG. 33 is a diagram showing the current density by voltage of the organic solar cell including Single molecule 4 prepared in Example 17.

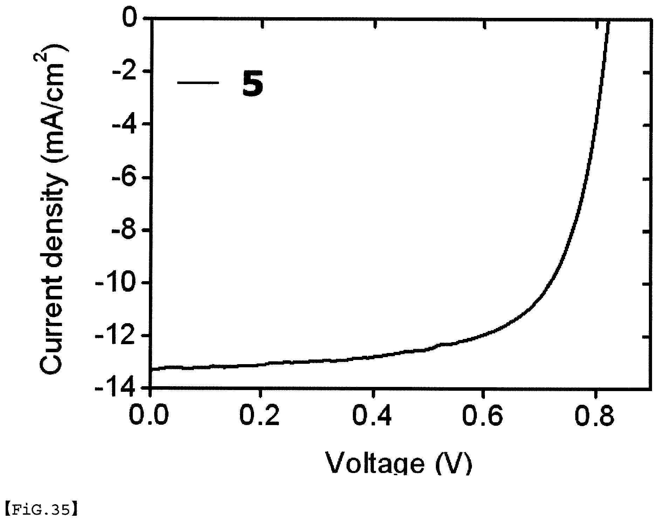

FIG. 34 is a diagram showing the current density by voltage of the organic solar cell including Single molecule 5 prepared in Example 18.

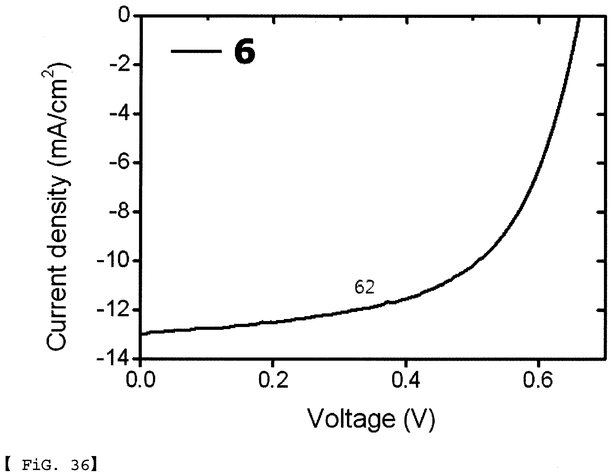

FIG. 35 is a diagram showing the current density by voltage of the organic solar cell including Single molecule 6 prepared in Example 19.

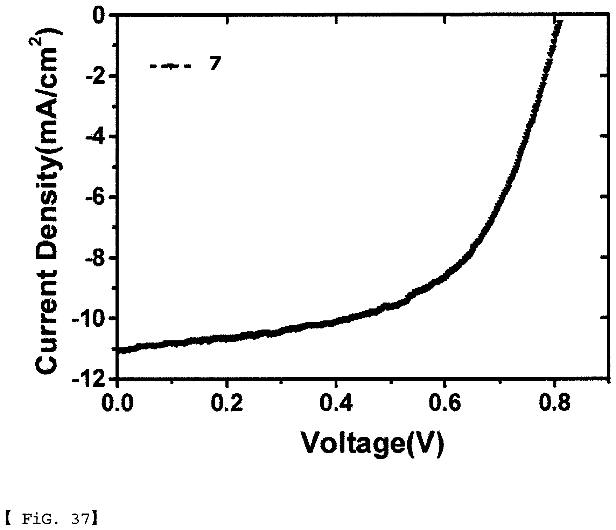

FIG. 36 is a diagram showing the current density by voltage of the organic solar cell including Single molecule 7 prepared in Example 20.

FIG. 37 is a diagram showing the current density by voltage of the organic solar cell according to Comparative Example 1.

## Claims

1. A single molecule which is represented by any one of the following Chemical Formulae 10, 11, 15, 16 and 18 to 21: 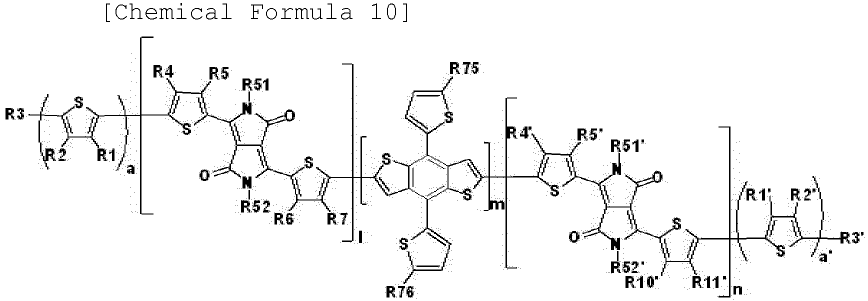 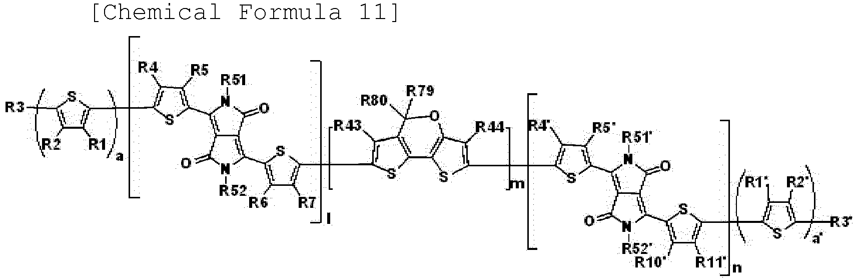 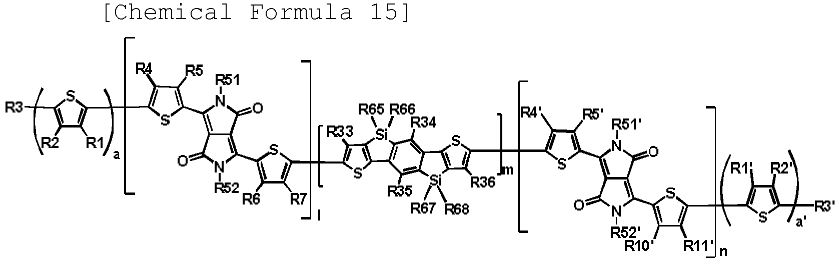 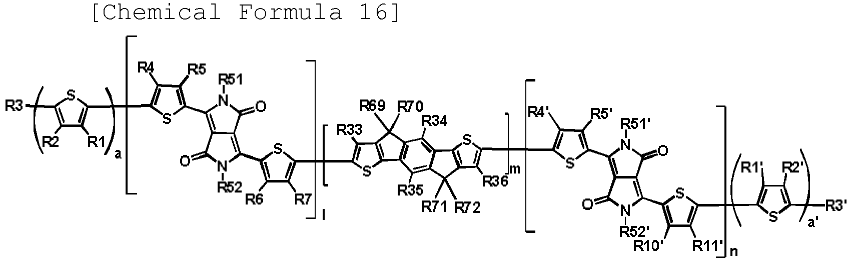 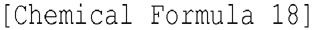 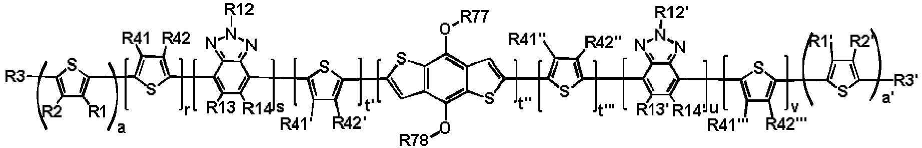 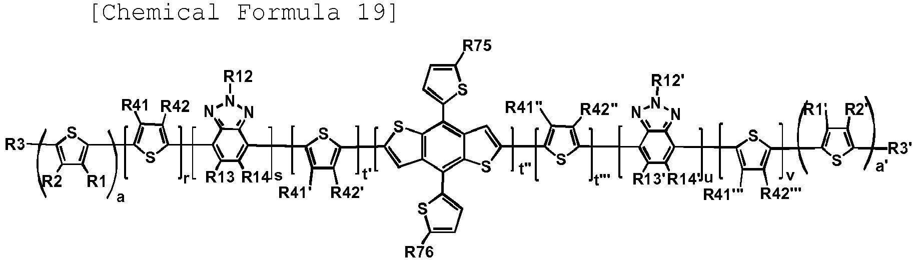 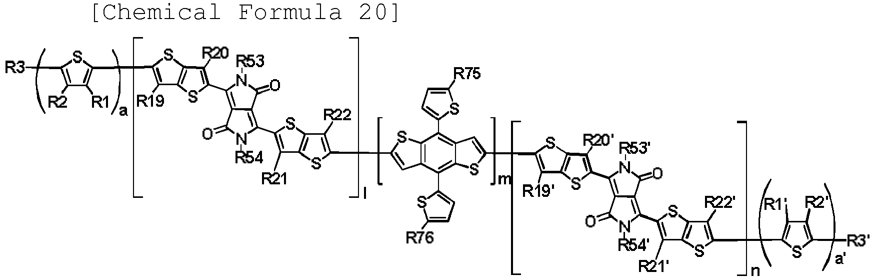 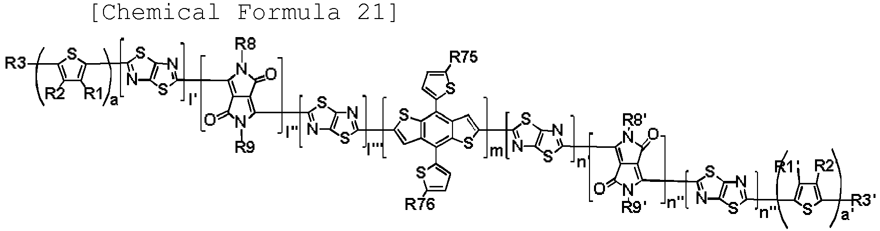 wherein, in Chemical Formulae 10, 11, 15, 16 and 18 to 21:
a is an integer of 1 to 5, and when a is an integer of 2 to 5, the two or more structures in the parenthesis are the same as or different from each other;
R1 and R2 are the same as or different from each other, and each independently selected from the group consisting of hydrogen; deuterium; a halogen group; a nitrile group; a nitro group; an imide group; an amide group; a hydroxyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; and a substituted or unsubstituted heteroring group;
R3 is hydrogen; a substituted or unsubstituted alkyl group; or any one of the following structures: 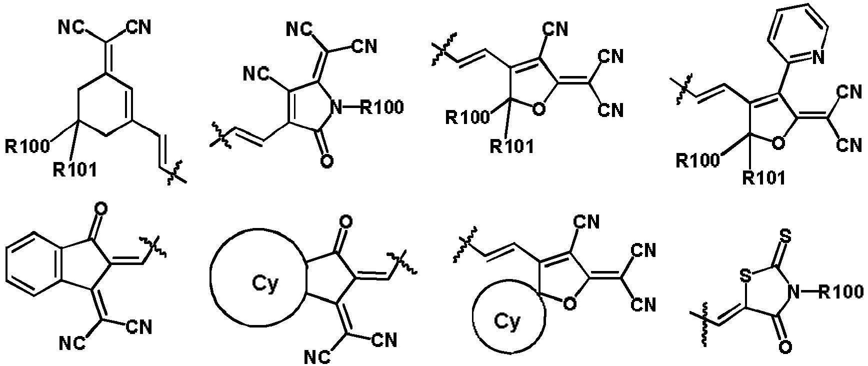 wherein, in the structure,
Cy is a substituted or unsubstituted aryl group; or a substituted or unsubstituted heteroring group;
R1 to R9, R12 to R14, R19 to R22, R33 to R36, R41 to R44, R51 to R54, R65 to R72, R75 to R80, R1' to R5', R8' to R14', R19' to R22', R41' to R42', R51' to R54', R41" to R42", R41'" to R42"', and R100, R101 are the same as or different from each other, and each independently selected from the group consisting of hydrogen; deuterium; a halogen group; a nitrile group; a nitro group; an imide group; an amide group; a hydroxyl group; a substituted or unsubstituted alkyl group; a substituted or unsubstituted cycloalkyl group; a substituted or unsubstituted alkoxy group; a substituted or unsubstituted aryloxy group; a substituted or unsubstituted alkylthioxy group; a substituted or unsubstituted arylthioxy group; a substituted or unsubstituted alkylsulfoxy group; a substituted or unsubstituted arylsulfoxy group; a substituted or unsubstituted alkenyl group; a substituted or unsubstituted silyl group; a substituted or unsubstituted boron group; a substituted or unsubstituted alkylamine group; a substituted or unsubstituted aralkylamine group; a substituted or unsubstituted arylamine group; a substituted or unsubstituted heteroarylamine group; a substituted or unsubstituted aryl group; and a substituted or unsubstituted heteroring group; and
a' has the same definition as a;
1 is a repetition number of units, and 0 ≤ 1 ≤ 10;
m is a repetition number of units, and 0 ≤ m ≤ 10;
n is a repetition number of units, and 0 ≤ n ≤ 10;
1+n ≥ 2;
2 ≤ 1+m+n ≤ 10;
l'+l"+l'''= l;
n'+n"+n'"=n;
t'+t"+t'"=t;
r is a repetition number of units, and 0 ≤ r ≤ 10;
s is a repetition number of units, and 0 ≤ s ≤ 10;
t is a repetition number of units, and 0 ≤ t ≤ 10;
u is a repetition number of units, and 2 ≤ u ≤ 10;
v is a repetition number of units, and 0 ≤ v ≤ 10;
s+u ≥ 2; and
2 ≤ r+s+t+u+v ≤ 10;
when 1, m, n, p, r, s, t, u and v each are two or more, the two or more structures of [ ] are the same as or different from each other.

2. The single molecule of Claim 1, which has a molecular weight of 100 g/mol to 20,000 g/mol.

3. An organic solar cell comprising:
a first electrode;
a second electrode provided opposite to the first electrode; and
one or more organic material layers provided between the first electrode and the second electrode, and including a photoactive layer,
wherein one or more layers of the organic material layers include the single molecule of Claim 1.

4. The organic solar cell of Claim 3, wherein the organic material layer includes a hole transfer layer, a hole injection layer, or a layer carrying out hole transfer and hole injection at the same time, and the hole transfer layer, the hole injection layer, or the layer carrying out hole transfer and hole injection at the same time includes the single molecule.

5. The organic solar cell of Claim 3, wherein the organic material layer includes an electron injection layer, an electron transfer layer, or a layer carrying out electron injection and electron transfer at the same time, and the electron injection layer, the electron transfer layer, or the layer carrying out electron injection and electron transfer at the same time includes the single molecule.

6. The organic solar cell of Claim 3, wherein the photoactive layer includes one, two or more selected from the group consisting of an electron donor and an electron acceptor, and the electron donor includes the single molecule.

## Patentansprüche

1. Einzelmolekül, das durch eine der nachfolgenden chemischen Formeln 10, 11, 15, 16 und 18 bis 21 dargestellt ist: 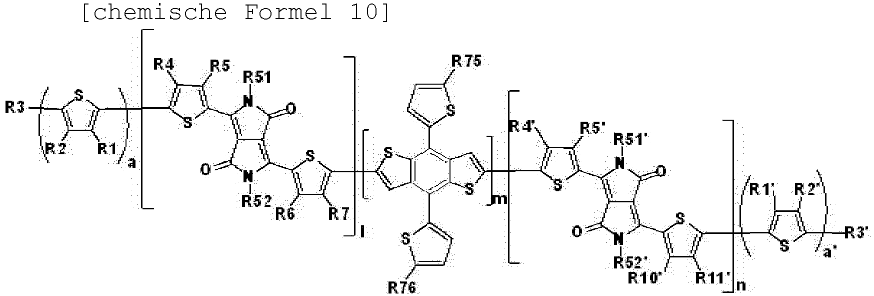 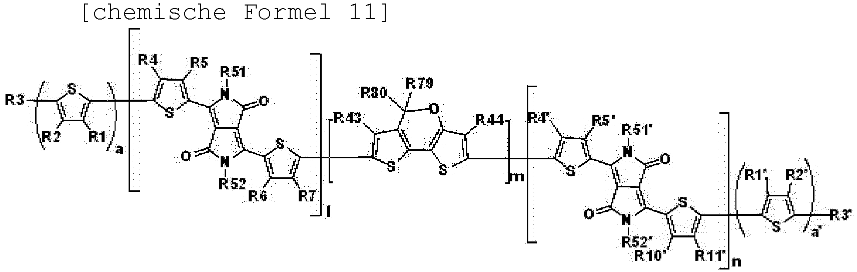 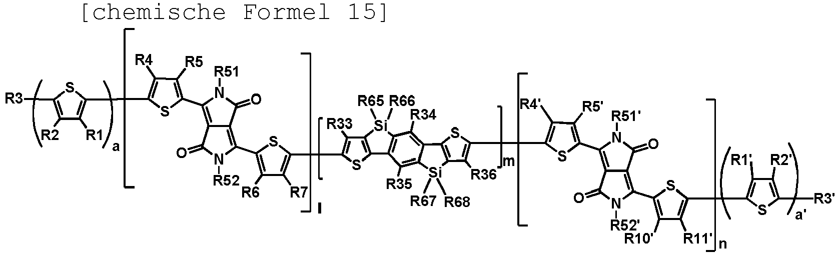 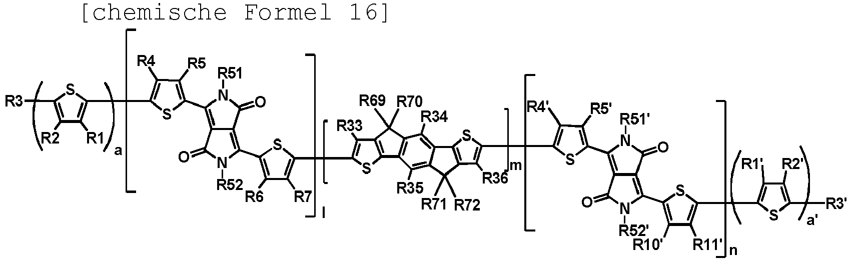 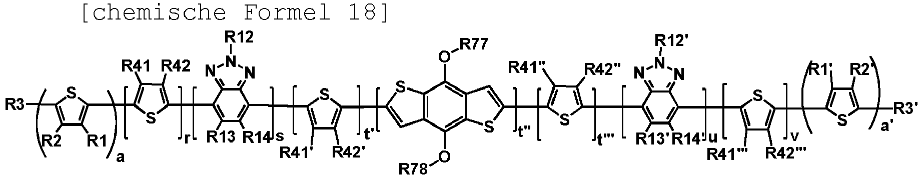 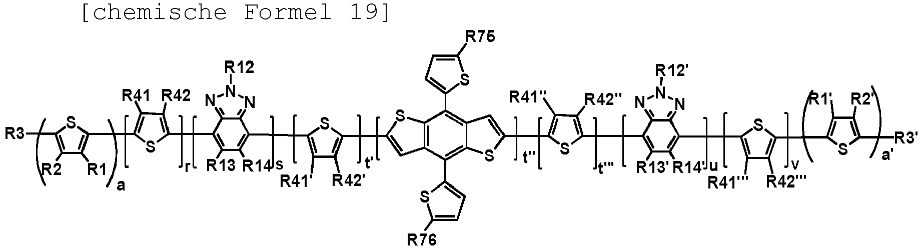 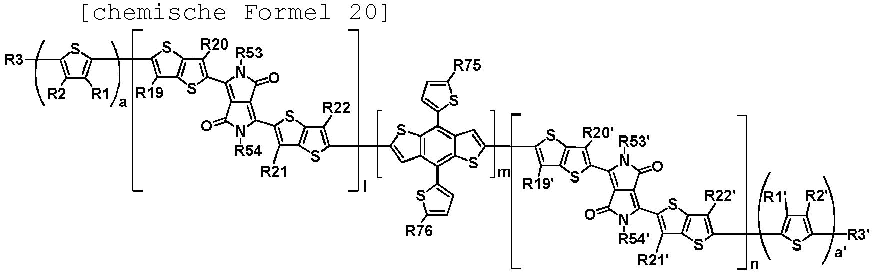 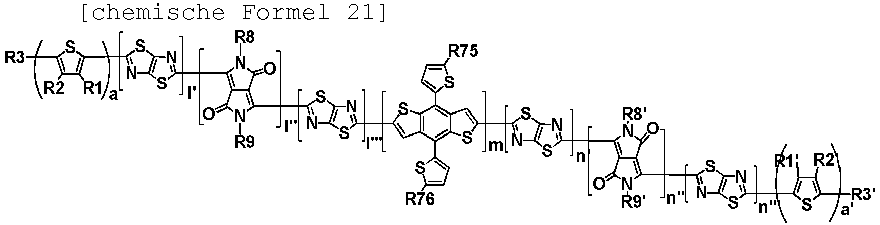 worin in den chemischen Formeln 10, 11, 15, 16 und 18 bis 21:
a eine ganze Zahl von 1 bis 5 ist, und wenn a eine ganze Zahl von 2 bis 5 ist, die zwei oder mehr Strukturen in der Klammer gleich sind oder sich voneinander unterscheiden;
R1 und R2 gleich sind oder sich voneinander unterscheiden und jeweils unabhängig ausgewählt sind aus der Gruppe, bestehend aus Wasserstoff; Deuterium; einer Halogengruppe; einer Nitrilgruppe; einer Nitrogruppe; einer Imidgruppe; einer Amidgruppe; einer Hydroxylgruppe; einer substituierten oder unsubstituierten Alkylgruppe; einer substituierten oder unsubstituierten Cycloalkylgruppe; einer substituierten oder unsubstituierten Alkoxygruppe; einer substituierten oder unsubstituierten Aryloxygruppe; einer substituierten oder unsubstituierten Alkylthioxygruppe; einer substituierten oder unsubstituierten Arylthioxygruppe; einer substituierten oder unsubstituierten Alkylsulfoxygruppe; einer substituierten oder unsubstituierten Arylsulfoxygruppe; einer substituierten oder unsubstituierten Alkenylgruppe; einer substituierten oder unsubstituierten Silylgruppe; einer substituierten oder unsubstituierten Borgruppe; einer substituierten oder unsubstituierten Alkylamingruppe; einer substituierten oder unsubstituierten Aralkylamingruppe; einer substituierten oder unsubstituierten Arylamingruppe; einer substituierten oder unsubstituierten Heteroarylamingruppe; einer substituierten oder unsubstituierten Arylgruppe; und einer substituierten oder unsubstituierten Heteroringgruppe;
R3 für Wasserstoff; eine substituierte oder unsubstituierte Alkylgruppe; oder eine der nachfolgenden Strukturen steht: 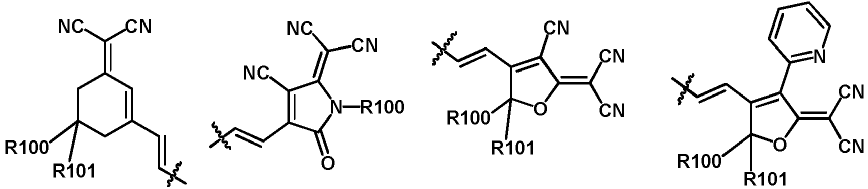 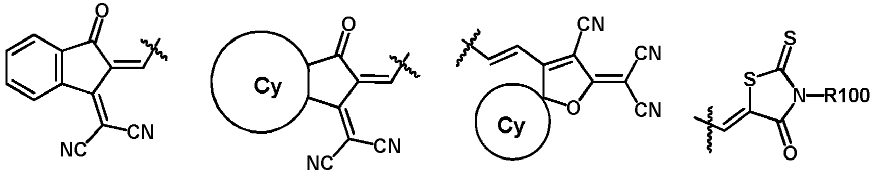 worin in der Struktur
Cy für eine substituierte oder unsubstituierte Arylgruppe; oder eine substituierte oder unsubstituierte Heteroringgruppe steht;
R1 bis R9, R12 bis R14, R19 bis R22, R33 bis R36, R41 bis R44, R51 bis R54, R65 bis R72, R75 bis R80, R1' bis R5', R8' bis R14', R19' bis R22', R41' bis R42', R51' bis R54', R41'' bis R42'', R41''' bis R42''' und R100, R101 gleich sind oder sich voneinander unterscheiden und jeweils unabhängig ausgewählt sind aus der Gruppe, bestehend aus Wasserstoff; Deuterium; einer Halogengruppe; einer Nitrilgruppe; einer Nitrogruppe; einer Imidgruppe; einer Amidgruppe; einer Hydroxylgruppe; einer substituierten oder unsubstituierten Alkylgruppe; einer substituierten oder unsubstituierten Cycloalkylgruppe; einer substituierten oder unsubstituierten Alkoxygruppe; einer substituierten oder unsubstituierten Aryloxygruppe; einer substituierten oder unsubstituierten Alkylthioxygruppe; einer substituierten oder unsubstituierten Arylthioxygruppe; einer substituierten oder unsubstituierten Alkylsulfoxygruppe; einer substituierten oder unsubstituierten Arylsulfoxygruppe; einer substituierten oder unsubstituierten Alkenylgruppe; einer substituierten oder unsubstituierten Silylgruppe; einer substituierten oder unsubstituierten Borgruppe; einer substituierten oder unsubstituierten Alkylamingruppe; einer substituierten oder unsubstituierten Aralkylamingruppe; einer substituierten oder unsubstituierten Arylamingruppe; einer substituierten oder unsubstituierten Heteroarylamingruppe; einer substituierten oder unsubstituierten Arylgruppe; und einer substituierten oder unsubstituierten Heteroringgruppe; und
a' die gleiche Definition wie a hat;
l eine Wiederholungszahl von Einheiten ist und 0 ≤ l ≤ 10;
m eine Wiederholungszahl von Einheiten ist und 0 ≤ m ≤ 10;
n eine Wiederholungszahl von Einheiten ist und 0 ≤ n ≤ 10;
1+n ≥ 2;
2 ≤ l+m+n ≤ 10;
l'+l"+l'"= l;
n'+n"+n'"= n;
t'+t"+t'"=t;
r eine Wiederholungszahl von Einheiten ist und 0 ≤ r ≤ 10;
s eine Wiederholungszahl von Einheiten ist und 0 ≤ s ≤ 10;
t eine Wiederholungszahl von Einheiten ist und 0 ≤ t ≤ 10;
u eine Wiederholungszahl von Einheiten ist und 2 ≤ u ≤ 10;
v eine Wiederholungszahl von Einheiten ist und 0 ≤ v ≤ 10;
s+u ≥ 2; und
2 ≤ r+s+t+u+v ≤ 10;
wenn l, m, n, p, r, s, t, u und v jeweils zwei oder mehr betragen, die zwei oder mehr Strukturen von [ ] gleich sind oder sich voneinander unterscheiden.

2. Einzelmolekül gemäß Anspruch 1, das ein Molekulargewicht von 100 g/mol bis 20.000 g/mol aufweist.

3. Organische Solarzelle, umfassend:
eine erste Elektrode;
eine zweite Elektrode, die gegenüber der ersten Elektrode bereitgestellt ist; und
ein oder mehrere der Schichten aus organischem Material, die zwischen der ersten Elektrode und der zweiten Elektrode bereitgestellt sind und eine photoaktive Schicht enthalten,
worin ein oder mehrere Schichten aus organischem Material das Einzelmolekül gemäß Anspruch 1 enthalten.

4. Organische Solarzelle gemäß Anspruch 3, worin die Schicht aus organischem Material eine Lochtransferschicht, eine Lochinjektionsschicht oder eine Schicht enthält, die gleichzeitig Lochtransfer und Lochinjektion ausführt, und die Lochtransferschicht, die Lochinjektionsschicht oder die Schicht, die gleichzeitig Lochtransfer und Lochinjektion ausführt, das Einzelmolekül enthält.

5. Organische Solarzelle gemäß Anspruch 3, worin die Schicht aus organischem Material eine Elektroneninjektionsschicht, eine Elektronentransferschicht oder eine Schicht enthält, die gleichzeitig Elektroneninjektion und Elektronentransfer ausführt, und die Elektroneninjektionsschicht, die Elektronentransferschicht oder die Schicht, die gleichzeitig Elektroneninjektion und Elektronentransfer ausführt, das Einzelmolekül enthält.

6. Organische Solarzelle gemäß Anspruch 3, worin die photoaktive Schicht eines, zwei oder mehrere enthält, ausgewählt aus der Gruppe, bestehend aus einem Elektronendonor und einem Elektronenakzeptor, und der Elektronendonor das Einzelmolekül enthält.

## Revendications

1. Molécule unique qui est représentée par l'une quelconque des formules chimiques 10, 11, 15, 16 et 18 à 21 suivantes : 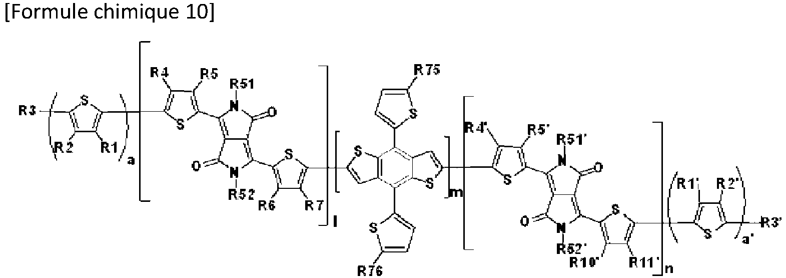 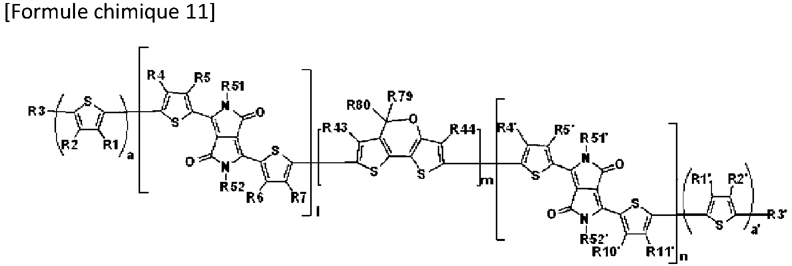 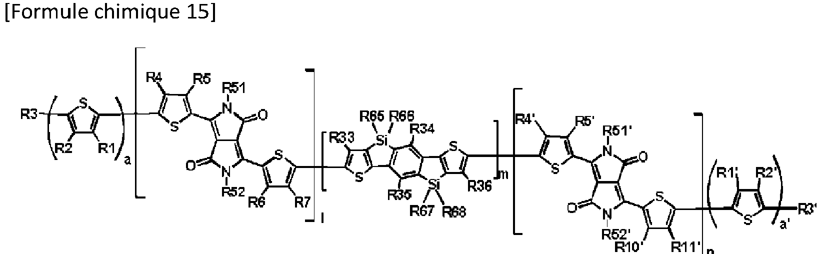 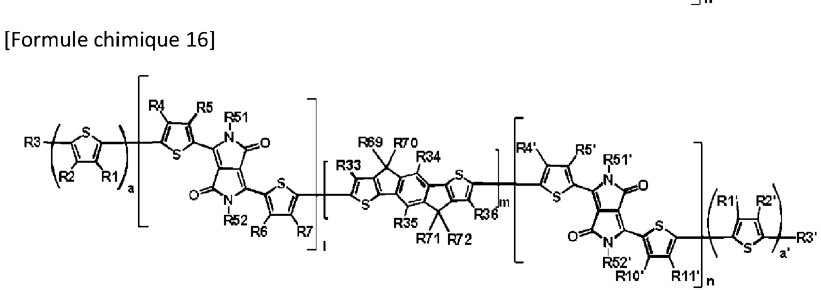 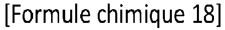 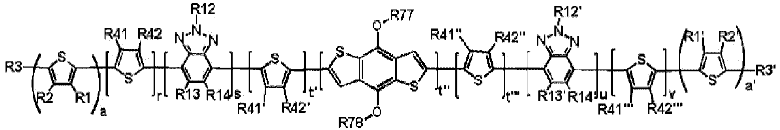 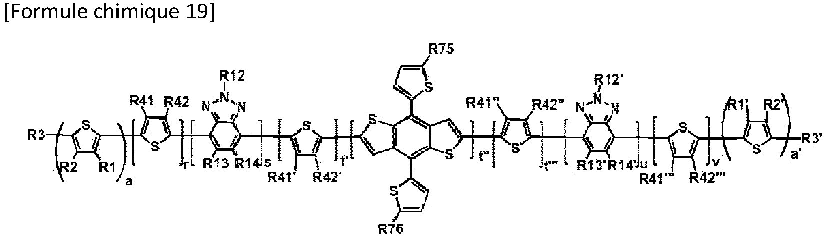 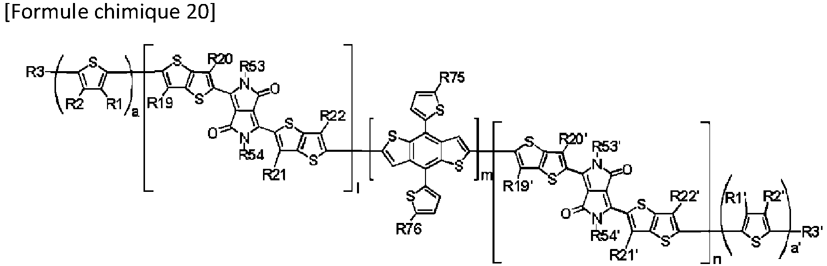 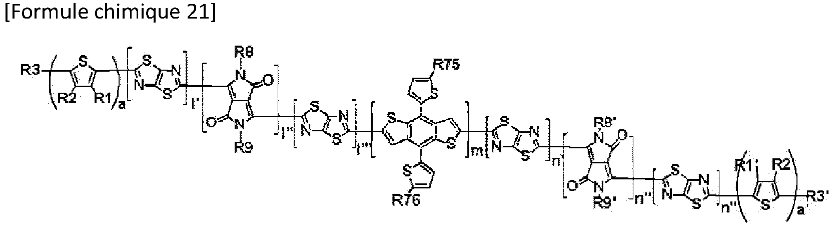 dans laquelle, dans les formules chimiques 10, 11, 15, 16 et 18 à 21 :
a est un entier de 1 à 5, et lorsque a est un entier de 2 à 5, les deux structures ou plus dans la parenthèse sont identiques ou différentes les unes des autres ;
R1 et R2 sont identiques ou différents l'un de l'autre, et chacun indépendamment choisi dans le groupe consistant en un hydrogène ; un deutérium ; un groupe halogène ; un groupe nitrile ; un groupe nitro ; un groupe imide; un groupe amide; un groupe hydroxyle ; un groupe alkyle substitué ou non substitué ; un groupe cycloalkyle substitué ou non substitué ; un groupe alcoxy substitué ou non substitué ; un groupe aryloxy substitué ou non substitué ; un groupe alkylthioxy substitué ou non substitué ; un groupe arylthioxy substitué ou non substitué ; un groupe alkylsulfoxy substitué ou non substitué ; un groupe arylsulfoxy substitué ou non substitué ; un groupe alcényle substitué ou non substitué ; un groupe silyle substitué ou non substitué ; un groupe bore substitué ou non substitué; un groupe alkylamine substitué ou non substitué; un groupe aralkylamine substitué ou non substitué ; un groupe arylamine substitué ou non substitué; un groupe hétéroarylamine substitué ou non substitué; un groupe aryle substitué ou non substitué ; et un groupe hétérocycle substitué ou non substitué ;
R3 est un hydrogène; un groupe alkyle substitué ou non substitué; ou l'une quelconque des structures suivantes : 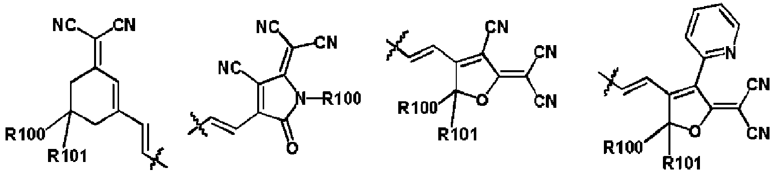 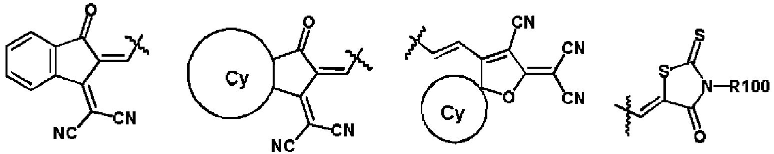 dans laquelle, dans la structure,
Cy est un groupe aryle substitué ou non substitué ; ou un groupe hétérocycle substitué ou non substitué ;
R1 à R9, R12 à R14, R19 à R22, R33 à R36, R41 à R44, R51 à R54, R65 à R72, R75 à R80, R1' à R5', R8' à R14', R19' à R22', R41' à R42', R51'à R54', R41" à R42", R41"' à R42'", et R100, R101 sont identiques ou différents les uns des autres,
et chacun indépendamment choisi dans le groupe consistant en un hydrogène; un deutérium ; un groupe halogène; un groupe nitrile ; un groupe nitro ; un groupe imide; un groupe amide; un groupe hydroxyle ; un groupe alkyle substitué ou non substitué ; un groupe cycloalkyle substitué ou non substitué ; un groupe alcoxy substitué ou non substitué ; un groupe aryloxy substitué ou non substitué ; un groupe alkylthioxy substitué ou non substitué ; un groupe arylthioxy substitué ou non substitué ; un groupe alkylsulfoxy substitué ou non substitué ; un groupe arylsulfoxy substitué ou non substitué ; un groupe alcényle substitué ou non substitué ; un groupe silyle substitué ou non substitué; un groupe bore substitué ou non substitué; un groupe alkylamine substitué ou non substitué; un groupe aralkylamine substitué ou non substitué; un groupe arylamine substitué ou non substitué ; un groupe hétéroarylamine substitué ou non substitué ; un groupe aryle et substitué ou non substitué ; et un groupe hétérocycle substitué ou non substitué ; et
a' présente la même définition que a ;
l est un nombre de répétitions d'unités, et 0 ≤ l ≤ 10 ;
m est un nombre de répétitions d'unités, et 0 ≤ m ≤ 10 ;
n est un nombre de répétitions d'unités, et 0 ≤ n ≤ 10 ;
l+n≥2;
2 ≤ l + m + n ≤ 10;
l' + l" + l'" = l ;
n' + n" + n'" = n ;
t' + t" + t'" = t ;
r est un nombre de répétitions d'unités, et 0 ≤ r ≤ 10 ;
s est un nombre de répétitions d'unités, et 0 ≤ s ≤ 10 ;
t est un nombre de répétitions d'unités, et 0 ≤ t ≤ 10 ;
u est un nombre de répétitions d'unités, et 0 ≤ u ≤ 10 ;
v est un nombre de répétitions d'unités, et 0 ≤ v ≤ 10 ;
s + u ≥ 2 ; et
2≤r+s+t+u+v≤10;
lorsque l, m, n, p, r, s, t, u et v sont chacun égaux ou supérieurs à deux, les deux structures ou plus de [ ] sont identiques ou différentes les unes des autres.

2. Molécule unique selon la revendication 1, qui présente un poids moléculaire de 100 g/mole à 20 000 g/mole.

3. Cellule solaire organique comprenant :
une première électrode ;
une seconde électrode disposée à l'opposé de la première électrode ; et
une ou plusieurs couches de matière organique disposées entre la première électrode et la seconde électrode, et incluant une couche photoactive,
dans laquelle une ou plusieurs couches des couches de matière organique incluent la molécule unique selon la revendication 1.

4. Cellule solaire organique selon la revendication 3, dans laquelle la couche de matière organique inclut une couche de transfert de trous, une couche d'injection de trous, ou une couche réalisant un transfert de trous et une injection de trous en même temps, et la couche de transfert de trous, la couche d'injection de trous, ou la couche réalisant un transfert de trous et une injection de trous en même temps inclut la molécule unique.

5. Cellule solaire organique selon la revendication 3, dans laquelle la couche de matière organique inclut une couche d'injection d'électrons, une couche de transfert d'électrons, ou une couche réalisant une injection d'électrons et un transfert d'électrons en même temps, et la couche d'injection d'électrons, la couche de transfert d'électrons, ou la couche réalisant une injection d'électrons et un transfert d'électrons en même temps inclut la molécule unique.

6. Cellule solaire organique selon la revendication 3, dans laquelle la couche photoactive inclut un, deux ou plus choisis dans le groupe consistant en un donneur d'électrons et un accepteur d'électrons, et le donneur d'électrons inclut la molécule unique.
